(19) Europäisches Patentamt
European Patent Office
Office européen des brevets

(11) **EP 2 110 124 B1**

(12) **EUROPEAN PATENT SPECIFICATION**

(45) Date of publication and mention
of the grant of the patent:
**17.07.2013 Bulletin 2013/29**

(51) Int Cl.:
*A61K 9/20* *(2006.01)* *A61K 31/421* *(2006.01)*
*A61P 21/00* *(2006.01)* *A61P 21/02* *(2006.01)*

(21) Application number: **09165833.6**

(22) Date of filing: **08.03.2005**

(54) **Bioavailable Solid Dosage Forms of Metaxalone**

Bioverfügbare feste Verabreichungsformen von Metaxalon

Formes galéniques de métaxalone solides et biodisponibles

(84) Designated Contracting States:
**AT BE BG CH CY CZ DE DK EE ES FI FR GB GR HU IE IS IT LI LT LU MC NL PL PT RO SE SI SK TR**

(30) Priority: **08.03.2004 US 551257 P**

(43) Date of publication of application:
**21.10.2009 Bulletin 2009/43**

(62) Document number(s) of the earlier application(s) in accordance with Art. 76 EPC:
**05732516.9 / 1 755 559**

(73) Proprietor: **Spireas, Spiridon**
**Newtown PA 18940 (US)**

(72) Inventor: **Spireas, Spiridon**
**Newtown PA 18940 (US)**

(74) Representative: **Clarke, Geoffrey Howard et al**
**avidity IP**
**Kestrel House**
**Falconry Court**
**Baker's Lane**
**Epping, Essex CM16 5DQ (GB)**

(56) References cited:
**WO-A-2004/019937     US-A- 3 993 767**
**US-A- 4 036 957     US-A- 5 800 834**
**US-B1- 6 407 128**

**Description**

FIELD OF THE INVENTION

The present invention relates to bioavailable pharmaceutical formulations of essentially water-insoluble drugs and processes for producing the same. In particular, the present invention relates to bioavailable, manufacturable, and stable pharmaceutical solid dosage forms comprising the essentially water-insoluble drug metaxalone, a muscle-relaxant, and processes for producing the same.

BACKGROUND OF THE INVENTION

Bioavailability of certain drugs, e.g., essentially water-insoluble drugs, can be limited when administered orally. Typically, manufacturers recommend that essentially water-insoluble drugs be taken along with food to enhance the bioavailability of the drugs.

For example, metaxalone or 5-[(3,5-dimethylphenoxy)methyl]-2 oxazolidinone is an essentially water-insoluble drug substance commercially available in the United States of America under the brand name skeliaxin® (trademark owned by Élan Pharmaceuticals, Inc. and distributed by King Pharmaceuticals), which is indicated as adjunct to rest, physical therapy and other measures for the relief of discomforts associated with acute. painful musculoskeletal conditions. Skelaxin® tablets containing 400 mg and 800 mg of metaxalone are presently available in the market.

Physical and chemical characteristics of metaxalone along with pharmacological, therapeutic and pharmacokinetic properties of the drug are reviewed in U.S. Patents No. 6,407,128 and 6,683,102. As discussed in those patents and relevant literature cited therein, metaxalone is a hydrophobic, essentially water-insoluble powder, which demonstrates limited absorption from the gastrointestinal tract (GIT) when administered orally in the form of Skelaxin® tablets containing 400 mg metaxalone and other inert compression tabletting excipients. The two patents present a method to improve the oral bioavailability of metaxalone from the Skelaxin® tablet formulation by administering Skelaxin® tablets with food.

Apparently, the poor bioavailability of metaxalone is due to its low aqueous solubility resulting into a slow rate of drug dissolution in the aqueous contents of the GIT. According to the two patents listed above, the aqueous solubility of metaxalone and, hence, its dissolution rate and oral bioavailability are not enhanced by the formulation used to produce the Skelaxin® tablets. Consequently, the oral bioavailability of metaxalone from Skelaxin® tablets is improved only when it is administered with food wherein the lipids and other fats and substances contained in the food along with the excessive presence of bile salts and digestive enzymes in the GIT caused by the food, act as solubility enhancers of metaxalone thereby increasing the drug dissolution rate and oral bioavailability of Skelaxin® tablets.

Therefore, it would be beneficial to the extent and rate of drug absorption and, in general, to the oral bioavailability of essentially water-insoluble drugs, e.g., metaxalone, if specialty formulations were introduced that would enhance the solubility and dissolution rate of the drug without the need of co-administration of food. Hence, there is presently a need for manufacturable and stable solid dosage forms of metaxalone, which would possess improved oral bioavailability and/or drug dissolution rates as compared to the commercially available Skelaxin® tablets. In addition, it is also desirable for one to be able to manufacture bioavailable pharmaceutical tablet formulations of metaxalone which would demonstrate improved drug dissolution rates as compared to those of Skelaxin® tablets while at the same time, possessing similar bioavailability properties to those of the commercially available product, i.e., being bioequivalent to the Skelaxin® tablets after single dose oral administration to fasting and/or non-fasting human subjects.

International patent application with publication number WO-2004/019937 and a publication date of March 11, 2004, deals with pharmaceutical compositions of metaxalone containing the drug in a micronized form wherein the metaxalone particles are reduced to levels below 10 micrometers ($\mu$m) of particle diameter. Specifically, 99% by volume of the particles possess particle diameters which are below a value of 10 $\mu$m. The authors of WO-2004/019937 suggest that their approach yields metaxalone 400-mg tablets with enhanced oral bioavailability as compared to commercially available Skelaxin® 400-mg tablets after single oral administration to nine (9) healthy male volunteers fasted overnight. However, the purported increase in oral bioavailability presented in that patent publication is marginal in terms of extent of absorption as defined by the Ln-transformed Test/Reference Ratio Percent of $AUC_{inf}$ (defined in a later section of this specification). As shown in Table 8 of WO-2004/019937, said Test/Reference $AUC_{inf}$ Ratio Percent did not even exceed the value of 125% which is widely accepted by regulatory agencies including the U.S. FDA as the upper limit of the 90% confidence intervals indicating significant difference between the oral bioavailability of the Test and Reference products. Furthermore, based on such results, the authors of WO-2004/019937 claim that using their micronized metaxalone tablet formulation would not present a food effect on oral bioavailability. However, the marginal increase in the Test/Reference $AUC_{inr}$ Ratio Percent observed in those studies implies exactly the opposite. In other words, it should be expected that, as reported in U.S. Patents No. 6,407,128 and 6,683,102 wherein the Skelaxin® reference tablets were dosed under fasting and non-fasting conditions, the metaxalone tablets of WO-2004/019937 which demonstrated an extent of absorption quite similar to that of the Skelaxin® 400-mg tablets under fasting dosing conditions, should also show a significant food effect on oral bioavailability which should be quite similar to that of the commercially available Skelaxin® 400-mg tablets.

As such, there remains a long-standing need for improved bioavailable formulations of essentially water-insoluble drugs and methods for preparation of the same. An object of the present invention is to provide pharmaceutical compositions comprising an essentially water-insoluble drug, metaxalone, and at least one inactive ingredient, which demonstrate improved drug dissolution rates as compared to the commercially available product of the same essentially water-insoluble drug, e.g., Skelaxin®). Another object is to provide pharmaceutical compositions comprising an essentially water-insoluble drug, metaxalone, and at least one inactive ingredient, which demonstrate improved drug dissolution rates and truly enhanced bioavailability properties as compared to the commercially available product of the same essentially water-insoluble drug, e.g., Skelaxin®. Furthermore, another object of the present invention is to provide pharmaceutical compositions comprising the essentially water-insoluble drug and at least one inactive ingredient, which demonstrate maximally enhanced bioavailability properties under fasting and non-fasting dosing conditions as compared to conventionally made formulations or the commercially available product of the same essentially water-insoluble drug. Finally, another object of this invention is to provide pharmaceutical compositions comprising the essentially water-insoluble drug and at least one inactive ingredient, which do not only demonstrate maximally enhanced bioavailability properties as compared to conventionally made formulations or the commercially available product of the same essentially water-insoluble drug, but they also present none or no significant food effect on the drug's oral bioavailability, i.e., they demonstrate similar bioavailability properties when dosed to fasting and non-fasting human subjects in contrast to conventional or commercial formulations of the drug.

SUMMARY OF THE INVENTION

Pharmaceutical compositions prepared according to the present invention comprise metaxalone, treated with various combinations of sparingly water-soluble or essentially water-insoluble, natural or synthetic hydrocolloids and polymers with the use of several volatile liquids and nonvolatile liquids, while at the same time demonstrating certain desirable drug dissolution rates and oral bioavailability.

The present invention provides a pharmaceutical solid dosage form comprising metaxalone, at least one powder excipient selected from a group comprising sparingly water-soluble or essentially water-insoluble, natural or synthetic hydrocolloids and polymers, and at least one non-volatile liquid,

prepared by a method comprising the steps of;

providing an effective amount of metaxalone;

providing at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;

dry-mixing the effective amount of metaxalone and the at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;

wetting the metaxalone/powder blend with the at least one non-volatile liquid and at least one volatile liquid to form a mix;

granulating the mix to form a granulation;

drying and milling the granulation;

mixing with at least one other inactive powder excipient

and compressing into tablets to produce the pharmaceutical solid dosage form;

wherein the at least one non-volatile liquid is selected from the group consisting of propylene glycol, glycerin, liquid polyethylene glycols, semisolid polyethylene glycols, pharmasolve, liquid polysorbates, semisolid polysorbates, liquid spans, semisolid spans, liquid cremophors, semisolid cremophors, liquid pluronics, semisolid pluronics, liquid myglyols, semisolid myglyols, non-volatile oils, vitamin-E, lecithin, or combinations thereof;

the at least one volatile liquid is selected from the group consisting of methanol, ethanol, acetone, water, or combinations thereof;

and wherein:

(i) 29% by weight or greater of said metaxolone is dissolved about 30 minutes after said dosage form is placed in a peak glass dissolution vessel filled with 1000 mL of purified water, maintained at 25°C and stirred at a paddle speed of 100 rpm using a USP Type II (paddle) apparatus; or

(ii) 40% by weight or greater of said metaxalone is dissolved about 30 minutes after said dosage form is placed in a standard glass dissolution vessel filled with 1000 mL of purified water, maintained at 35°C and stirred at a paddle speed of 100 rpm using a USP Type II (paddle) apparatus; or

(iii) 30% by weight or greater of said metaxalone is dissolved about 30 minutes after said dosage form is placed in a peak glass dissolution vessel filled with 500 mL of an aqueous solution of 0.1% by weight of Sodium Lauryl Sulfate per volume of water, maintained at 37°C and stirred at a paddle speed of 50 rpm using a USP Type II (paddle) apparatus.

The present invention also provides a method of making a pharmaceutical solid dosage form according to any one of the appended claims comprising:

> providing an effective amount of metaxalone;
> providing at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;
> dry-mixing the effective amount of metaxalone and the at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;
> wetting the metaxalone/powder blend with at least one non-volatile liquid and at least one volatile liquid to form a mix;
> granulating the mix to form a granulation;
> drying and milling the granulation;
> mixing with at least one other powder excipient;
> and compressing into tablets to produce the pharmaceutical solid dosage form;
> wherein the at least one non-volatile liquid is selected from the group consisting of propylene glycol, glycerin, liquid polyethylene glycols, semisolid polyethylene glycols, pharmasolve, liquid polysorbates, semisolid polysorbates, liquid spans, semisolid spans, liquid cremophors, semisolid cremophors, liquid pluronics, semisolid pluronics, liquid myglyols, semisolid myglyols, non-volatile oils, vitamin-E, lecithin, or combinations thereof; and
> the at least one volatile liquid is selected from the group consisting of methanol, ethanol, acetone, water, or combinations thereof.

The practice of the present invention employs, unless otherwise indicated, conventional methods of chemistry and drug synthesis and formulation, all within the skill of the art. Such techniques are explained fully in the literature. See, e.g., Remington: The Science and Practice of Pharmacy by Alfonso R. Gennar, editor, (19th edition 2000).

BRIEF DESCRIPTION OF THE DRAWINGS

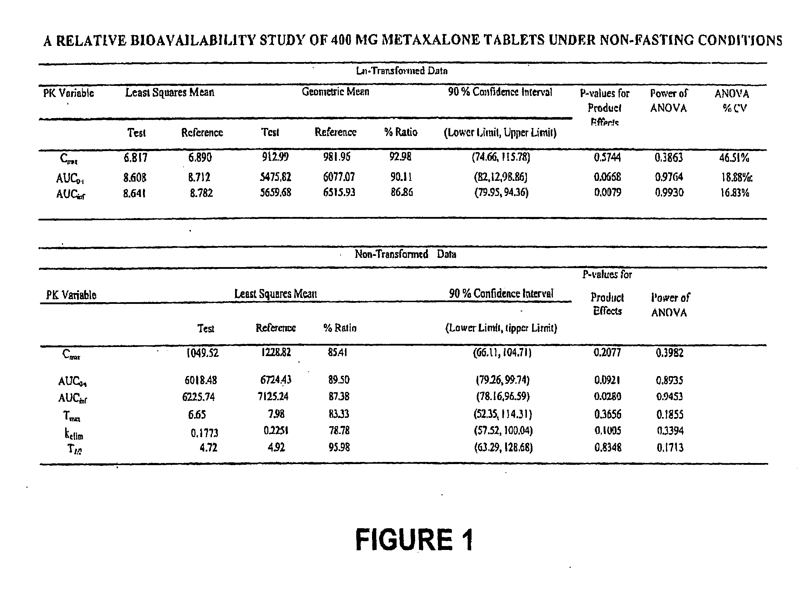

Figure 1 shows the summary of statistical analysis of dosing of metaxalone under non-fasting conditions in the forms of the Ex.#2 metaxalone 400-mg tablets (Lot # BB5800087) dosed as the Test product and commercial Skelaxin® 400-mg tablets (Lot # GS1109A) dosed as the Reference product.

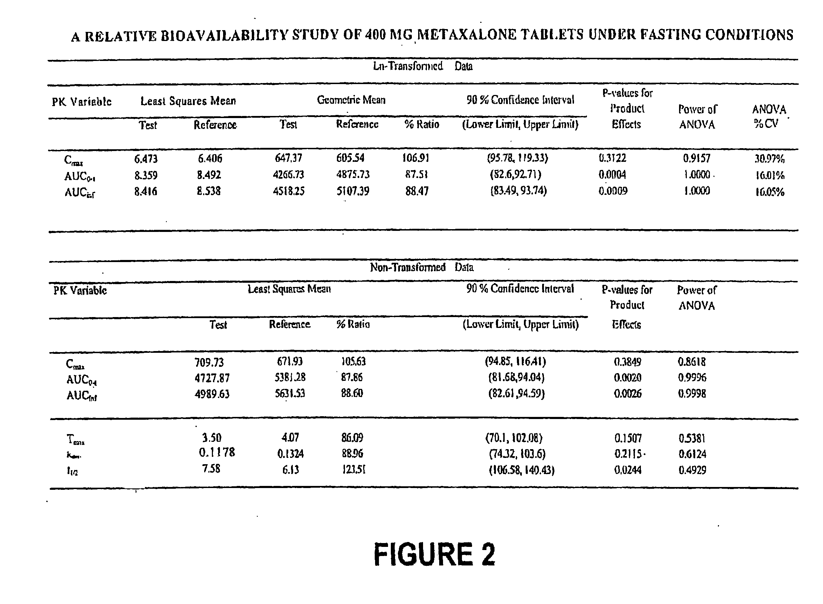

Figure 2 shows the summary of statistical analysis of dosing of metaxalone under fasting conditions in the forms of the Ex.#2 metaxalone 400-mg tablets (Lot # BB5800087) dosed as the Test product and Skelaxin® 400-mg tablets (Lot # GS1109A) dosed as the Reference product.

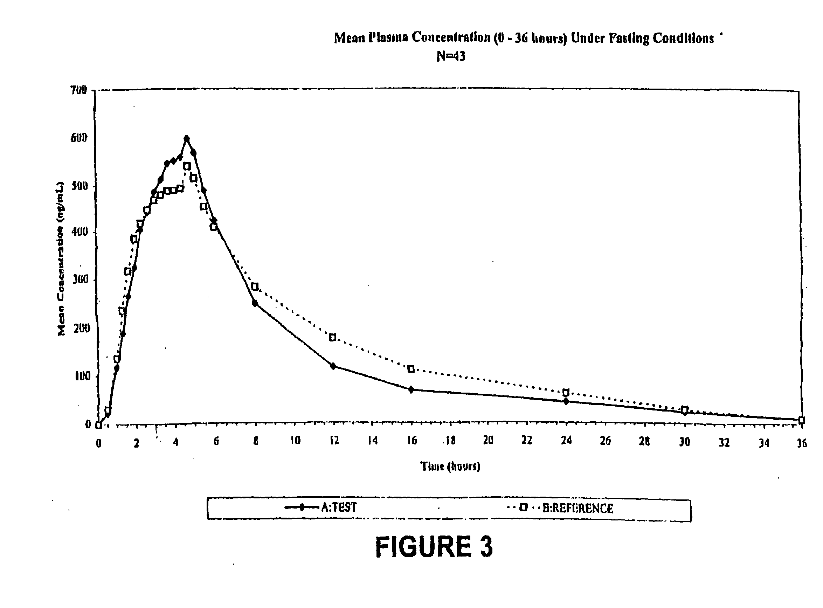

Figure 3 shows the mean plasma concentration (ng/mL) from time 0 hours to time 36 hours after dosing of metaxalone under fasting conditions in the forms of the Ex.#2 metaxalone 400-mg tablets (Lot # BB5800087) dosed as the Test product and commercial Skelaxin® 400-mg tablets (Lot # GS 1109A) dosed as the Reference product.

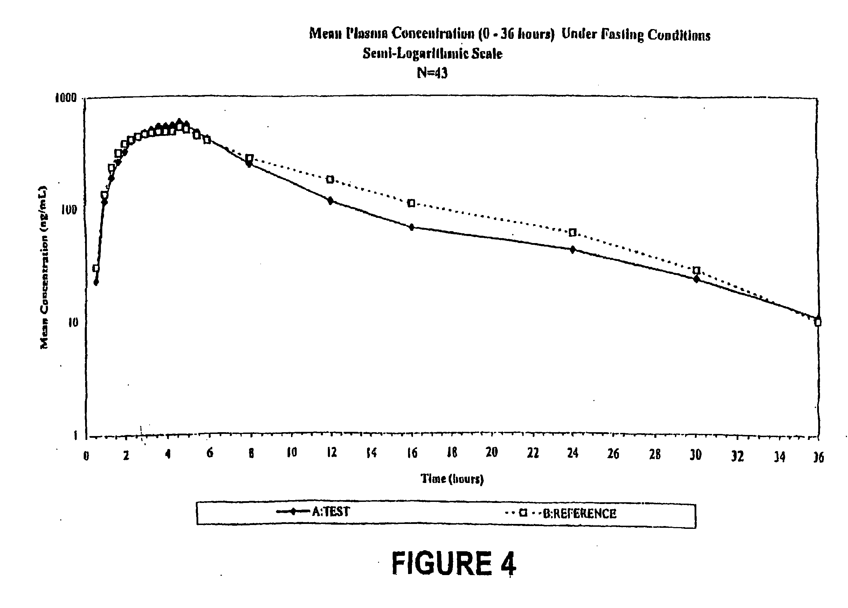

Figure 4 shows the mean plasma concentration (ng/mL) from time 0 hours to time 36 hours, on a semi-logarithmic scale, after dosing of metaxalone under fasting conditions in the forms of the Ex.#2 metaxalone 400-mg tablets (Lot # BB5800087) dosed as the Test product and commercial Skelaxin® 400-mg tablets (Lot # GS 1109A) dosed as the Reference product.

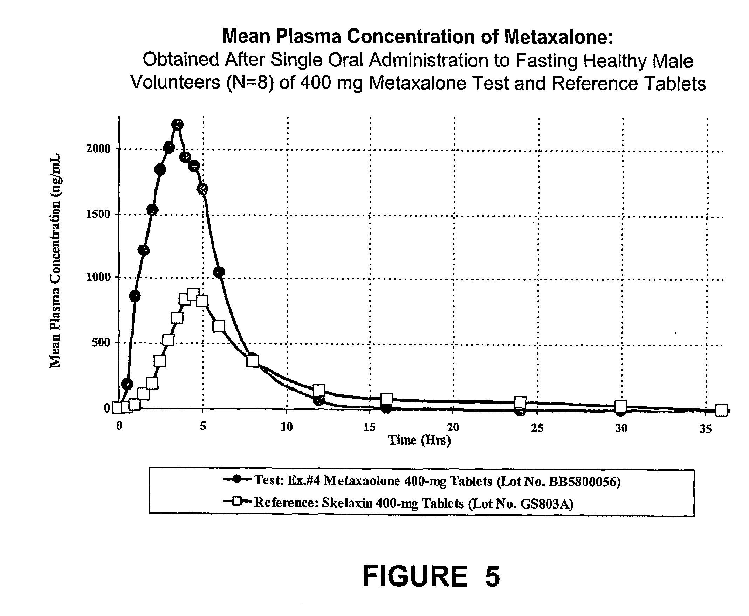

Figure 5 shows the mean plasma concentration (np/mL) from time 0 hours to time 36 hours after dosing of metaxalone under fasting conditions in the forms of the novel, maximally bioavailable Ex.#4 metaxalone 400-mg tablets (Lot # BB5800056) dosed as the Test product and commercial Skelaxin® 400-mg tablets (Lot # GS803A) dosed as the Reference product.

DETAILED DESCRIPTION OF ILLUSTRATIVE EMBODIMENTS

The present invention provides a pharmaceutical solid dosage form comprising metaxalone, at least one powder excipient selected from a group comprising sparingly water-soluble or essentially water-insoluble, natural or synthetic hydrocolloids and polymers, and at least one non-volatile liquid,
prepared by a method comprising the steps of;

providing an effective amount of metaxalone;

providing at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;

dry-mixing the effective amount of metaxalone and the at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;

wetting the metaxalone/powder blend with the at least one non-volatile liquid and at least one volatile liquid to form a mix;

granulating the mix to form a granulation;

drying and milling the granulation;

mixing with at least one other inactive powder excipient

and compressing into tablets to produce the pharmaceutical solid dosage form;

wherein the at least one non-volatile liquid is selected from the group consisting of propylene glycol, glycerin, liquid polyethylene glycols, semisolid polyethylene glycols, pharmasolve, liquid polysorbates, semisolid polysorbates, liquid spans, semisolid spans, liquid cremophors, semisolid cremophors, liquid pluronics, semisolid pluronics, liquid myglyols, semisolid myglyols, non-volatile oils, vitamin-E, lecithin, or combinations thereof;

the at least one volatile liquid is selected from the group consisting of methanol, ethanol, acetone, water, or combinations thereof;

and wherein:

(i) 29% by weight or greater of said metaxolone is dissolved about 30 minutes after said dosage form is placed in a peak glass dissolution vessel filled with 1000 mL of purified water, maintained at 25°C and stirred at a paddle speed of 100 rpm using a USP Type II (paddle) apparatus; or

(ii) 40% by weight or greater of said metaxalone is dissolved about 30 minutes after said dosage form is placed in a standard glass dissolution vessel filled with 1000 mL of purified water, maintained at 35°C and stirred at a paddle speed of 100 rpm using a USP Type II (paddle) apparatus; or

(iii) 30% by weight or greater of said metaxalone is dissolved about 30 minutes after said dosage form is placed in a peak glass dissolution vessel filled with 500 mL of an aqueous solution of 0.1% by weight of Sodium Lauryl Sulfate per volume of water, maintained at 37°C and stirred at a paddle speed of 50 rpm using a USP Type II (paddle) apparatus.

The present invention also provides a method of making a pharmaceutical solid dosage form according to any one of the appended claims comprising:

providing an effective amount of metaxalone;

providing at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;

dry-mixing the effective amount of metaxalone and the at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;

wetting the metaxalone/powder blend with at least one non-volatile liquid and at least one volatile liquid to form a mix;

granulating the mix to form a granulation;

drying and milling the granulation;

mixing with at least one other powder excipient;

and compressing into tablets to produce the pharmaceutical solid dosage form;

wherein the at least one non-volatile liquid is selected from the group consisting of propylene glycol, glycerin, liquid polyethylene glycols, semisolid polyethylene glycols, pharmasolve, liquid polysorbates, semisolid polysorbates, liquid spans, semisolid spans, liquid cremophors, semisolid cremophors, liquid pluronics, semisolid pluronics, liquid myglyols, semisolid myglyols, non-volatile oils, vitamin-E, lecithin, or combinations thereof; and

the at least one volatile liquid is selected from the group consisting of methanol, ethanol, acetone, water, or combinations thereof.

Overall, the present invention relates to compositions of metaxalone, which, in contrast to commercial formulations, e.g., the Skelaxin® tablets, demonstrate improved "drug dissolution rate" and/or an "oral bioavailability" equivalent to, or greater than, that of the commercially available tablet formulations. Skelaxin® (400 mg strength) was been approved by the FDA under NDA #13-217 prior to January 1, 1982. In 2002, supplemental new drug applications NDA #13-217/S-044 (400 mg) and NDA#13-2171S-036 (800 mg), which relate to administering Skelaxin® with food, were approved.

The term "drug dissolution rate" is defined herein as the amount or percent by weight of an essentially water-insoluble drug, e.g., metaxalone, dissolved within a given time period, namely, within the first 30 minutes of dissolution, from a unit of a solid dosage form, for example, from one tablet containing 400 mg of metaxalone being subjected to a specific dissolution test, which is characterized by a certain volume and type of the employed dissolution liquid medium that was maintained at a certain temperature and agitated by a given speed and type of a certain steering device. In the present studies, three dissolution tests (i.e., Tests A, B and C) were used to assess and compare the dissolution properties of the products evaluated. Such dissolution tests are described in a later section of this specification.

The term "oral bioavailability" is defined herein as the measure of the rate and extent of drug absorption in healthy human volunteers as expressed by $C_{max}$, which is the average maximum metaxalone concentration in plasma obtained during each study from human subjects, and $AUC_{inf}$, which is the average area under the metaxalone plasma concentration over time curve, obtained after a single dose oral administration of a drug product, for example, 400-mg metaxalone tablets (Skelaxin® or experimental tablets) during two period crossover clinical studies under fasting or non-fasting conditions. For the work related to the present invention, several two-period crossover relative bioavailability studies using various numbers of subjects ranging from 43 to 8 healthy male volunteers were conducted to compare the oral bioavailability of Skelaxin® reference tablets to that of experimental metaxalone test tablets. The methodology and statistical analysis employed in each of these biostudies are described in detail in a later section of this specification. Overall, in each of the biostudies performed, a given Test product (i.e., experimental metaxalone 400-mg tablets) was compared to the Reference product (i.e., commercial Skelaxin® 400-mg tablets) by using ANOVA treatments to assess the geometric means and upper and lower 90% confidence interval (CI) limits of the individual Test/Reference ratio percents of $AUC_{inf}$ and $C_{max}$ based on non-transformed and In-transformed drug plasma concentrations obtained at various time intervals after single oral administration of a given Test or Reference product to each subject.

It should be specified that, in the work related to this invention, a given experimental metaxalone tablet formulation tested, was considered to be "bioequivalent" to the reference Skelaxin® tablets of NDA #13-217 if both of the obtained In-transformed mean Test/Reference $AUC_{inf}$ and $C_{max}$ ratio percents along with their corresponding lower and upper CI limits were within a lower limit of 80% and an upper limit of 125%. On the other hand, experimental metaxalone products presenting both non-transformed and In-transformed mean Test/Reference $C_{max}$, $AUC_{0-1}$ and $AUC_{inf}$ ratio percents greater than an upper limit of 125% were considered to be "more bioavailable" than the reference Skelaxin® tablets of NDA #13-217. In addition, in the case that experimental metaxalone tablet formulations presented both non-transformed and In-transformed mean Test/Reference $C_{max}$. $AUC_{0-1}$ and $AUC_{inf}$ ratio percents greater than 150%, such products were considered to be "significantly more bioavailable" than the reference Skelaxin® tablets of NDA #13-217. Such "significantly more bioavailable" experimental metaxalone tablet formulations are unique according to the present invention and they are also referred to herein as "maximally bioavailable" compositions. Finally, In-transformed mean Test/Reference $AUC_{inf}$ and/or $C_{max}$ ratio percents smaller than the lower CI limit of 80% led to concluding that such tested metaxalone tablets were "less bioavailable" than the Skelaxin® tablets of NDA #13-217.

Based on the metaxalone tablet formulations developed in connection to this invention, it has been now discovered that pharmaceutical compositions containing metaxalone treated with various combinations of sparingly water-soluble or essentially water-insoluble, natural or synthetic hydrocolloids and polymers with the use of various volatile and nonvolatile liquids, can be prepared demonstrating significantly improved oral bioavailability and/or drug dissolution rates of metaxalone as compared to the commercial skelaxin® tablets.

As specified by the claims, hydrocolloids and polymers to be employed in the metaxalone solid dosage forms according to the present invention are selected from the group consisting of sodium alginate, ammonium alginates, sodium carboxymethyl cellulose, microcrystalline cellulose, pregelatinised starch, sodium starch glycolate, methacrylic acid copolymers or combinations thereof.

As specified by the claims, volatile liquids to be used in preparing the metaxalone solid dosage forms according to the present invention are selected from the group consisting of methanol. ethanol, acetone, water, or combinations thereof. As specified by the claims, nonvolatile liquids to be used in preparing the metaxalone solid dosage forms according to the present invention are selected from the group consisting of liquid or semisolid polyethylene glycols, propylene glycol glycerin, pharmasolve, liquid or semisolid polysorbates, liquid or semisolid cremophors, liquid or semisolid spans, liquid or semisolid myglyols, liquid or semisolid pluronics and other oils and oily liquids such as fish oil, olive oil, castor oil, vitamin-E, lecithin, or combinations thereof that may improve the aqueous solubility of metaxalone and/or the drug's wetting properties.

As used herein, the term "nonvolatile liquid" means any liquid which, at sea level and standard pressure conditions (i.e., atmospheric pressure equal to 1 atm) possesses a boiling point greater than the boiling point of distilled water, namely, a boiling point greater than 100°C. On the other hand, the term volatile liquid means any liquid which at sea level and at 1 atm pressure possesses a boiling point equal to or less than 100°C. Those of skill in the art will understand that whenever a volatile liquid is used to wet a powder admixture serving to promote a granulation process, it would be subsequently removed from the final granulation powder blend by means of drying. This, however, does not occur when nonvolatile liquids are incorporated into the formulation. In such case, the nonvolatile liquid does not evaporate during drying and it remains within the final dosage form for the life of the products. Pharmaceutical compositions containing nonvolatile liquids are termed Liquisolid Systems and are described in U.S. Patents No. 5,800,834, No. 5,968,550, No. 6,096,337 and No. 6,423,339.

Other than the ingredients discussed above, metaxalone compositions according to the present invention also contain, as specified by the claims, various inactive pharmaceutical excipients. Those of skill in the art will understand that the term "excipient" is used colloquially to include such pharmaceutical adjuvants as fillers, diluents, binders, disintegrants, glidants, lubricants, pigments, colorants, coating agents, lubricants and the like. "Fillers" and "diluents" are powders used to add volume to the drug/powder blends in order to improve the distribution, uniformity, stability and processing of the drug in the final product. "Hinders" are agents that hold the components of the formulation together. "Disintegrants" are agents that enhance the conversion of a compact material into fine primary particles during the dissolution of the final product. "Glidants" are additives that reduce particle friction and induce good flowing properties of the final drug/powder blend. "Lubricants" are additives used to prevent the sticking of the product formulation to tooling during the tabletting process. It will be appreciated by persons of ordinary skill in the art that such inactive pharmaceutical materials should be consistent with the overall spirit of the invention. Thus, such materials may be employed which do not adversely effect the processing set forth herein and which do not interfere with the stability of the resulting products.

Therefore, the term "excipient." "powder excipient," or "inactive powder excipient" as used herein include, but is not limited to: "hydrocolloids and polymers" discussed previously such as the "alginate powder excipients" (i.e., alginic acid and sodium, ammonium and calcium alginates), cellulosic derivatives, copolymers of acrylic acid, polyvinyl pyrrolidones and other natural, semi-synthetic or synthetic hydrocolloids and polymers; "cellulosic powder excipients" such as microcrystalline cellulose, powder and amorphous cellulose, carboxymethyl and sodium carboxymethyl cellulose, methyl and ethyl cellulose, hydroxyethyl cellulose and hydroxypropyl cellulose, hydroxypropylmethyl cellulose and other cellulosic derivatives and modified celluloses; "starch powder excipients" such as corn starch, maze starch, potato starch, pregelatinized starch, sodium starch glycolate and other starch derivatives; "sugar-base powder excipients" such as lactose, sucrose, maltose, dextrose, fructose, cyclodextrins, etc.; and "other powder adjuvants" such as dicalcium and tricalcium phosphate, amorphous silicon dioxide, fumed silica, talc, croscaramelose sodium, povidone, crosslinked povidone, magnesium stearate, stearic acid, glyceryl monostearate, hydrogenated vegetable oil, waxes and other inert powders including stabilizing, coloring and coating agents, that are useful for tabletting or encapsulation.

By "pharmaceutically acceptable" or "pharmacologically acceptable" is meant a material which is not biologically or otherwise undesirable, i.e., the material can be administered to an individual without causing any undesirable biological effects or interacting in a deleterious manner with any of the components of the formulation in which it is contained.

According to the invention as specified by the claims, the drug is dry-mixed with certain inactive and pharmaceutically acceptable powder excipients. The drug/powder blend is then wetted and granulated with certain pharmaceutically acceptable volatile liquids with the presence of certain pharmaceutically acceptable nonvolatile liquids. The wet granulation is then dried, milled and subsequently mixed with other powder excipients to yield the final drug/powder blend which is then compressed into tablets to produce the pharmaceutical composition or final solid dosage form.

As used herein, the term "drying" refers to the substantial removal of the volatile granulating liquid from the granulation. Drying may be accomplished in a number of manners well known to those of skill in the art including, but not limited to the use of ovens, fluid bed driers, and other similar equipment. In a preferred embodiment, the granulation is dried in an oven for about 8 hours at 50°C to substantially remove the volatile liquid from the granulation. In another preferred embodiment, the granulation is dried in an oven for about 10 hours at 50°C to substantially remove the volatile liquid from the granulation.

The processes of mixing, granulating, drying, milling, compressing, coating and making pharmaceutical solid formulations are well known to those of skill in the art. See, e.g., Theory & Practice of Industrial Pharmacy, 3rd Edition, Liberman, Lachman, and Kanig, eds. (Philadelphia, Pennsylvania: Lea & Febiger).

In this work, several metaxalone tablet formulations were prepared and tested for their drug dissolution rates using three different dissolution tests discussed below. Furthermore, four of the experimental metaxalone products were tested by conducting relative bioavailability studies using commercially available Skelaxin 400-mg tablets as the reference product. The bioavailability properties of such Test metaxalone products where compared to the Reference Skelaxin® tablets using various numbers of healthy male volunteers in single dose two-period crossover studies under fasting and non-fasting dosing conditions as discussed below.

Example Formulations: The experimental metaxalone tablet formulations prepared and tested are listed in Table 1. In general, metaxalone formulations were made by dry-mixing metaxalone powder with inactive powdered ingredients. Then, the resulting powder blends were wet-granulated using various volatile liquids with or without the presence of nonvolatile liquids. After drying and milling the granulation, other powder excipients were blended with the granulated powder to produce the final metaxalone product blend which was compressed into tablets each containing 400-mg of metaxalone. It should be noted that when nonvolatile liquids were incorporated in the final dosage forms as in the experimental metaxalone formulations Ex.#4 and Ex.#5 shown in Table 1 below, techniques and compositions related to the principles of liquisolid technology and systems were employed. Liquisolid systems and methods of producing same are discussed in U.S. Patents No. 5,800,834, No. 5,968,550, No. 6,096,337 and No. 6,423,339. The ingredients of the experimental metaxalone tablet formulations prepared herein are listed in Table 1.

**Table 1:** Ingredients and Other Characteristics of Prepared Experimental Metaxalone Tablet Formulations (Ex. #1-5).

| Formulation Ingredients (mg/tablet) | EXPERIMENTAL METAXALONE TABLET FORMULATIONS | | | | |
|---|---|---|---|---|---|
| | Ex. # 1 (BB5800040) | Ex. # 2 (BB5800087) | Ex. # 3 (BB5800047) | Ex. # 4 (BB5800056) | Ex. # 5 (NB1190:48) |
| Metaxalone (non-micronized) | 400 | 400 | 400 | 400 | 400 |
| Sodium Carboxy-Methyl Cellulose | 10 | - | 15 | 39 | 15 |
| Methacrylic Acid Copolymer | - | - | - | 25 | - |
| Ammonium Alginate | - | 21.5 | - | - | - |
| Sodium Alginate | - | 3 | - | - | - |
| Polyethylene Glycol 400 | - | - | - | 2 | - |
| Polysorbate 80 | - | - | - | - | 5 |
| Microcrystalline Cellulose | 65 | 30 | 66 | 33.5 | 66 |
| Pregelatinized Starch | 13.5 | 35 | 5.5 | - | 5.5 |
| Sodium Starch Glycolate | 7 | - | 4 | 4 | 4 |
| Magnesium Stearate | 4 | - | 4 | 4 | 4 |
| Stearic Acid | - | 4 | - | - | - |
| Red Iron Oxide | 0.5 | 0.5 | 0.5 | 0.5 | 0.5 |
| Denatured Alcohol (granulation liquid - not present in the final product) | - | (14) | (35) | (79) | (35) |
| Purified Water (granulation liquid- not present in the final product) | (130) | (130) | (155) | (15) | (155) |
| Tablet Weight | 500 | 494 | 495 | 508 | 500 |

**Dissolution Studies:** Three different dissolution tests (Test-A, -B and -C) based on a modified USP dissolution apparatus II (paddle method) were used to assess the drug dissolution profiles of each of the experimental metaxalone 400-mg tablet formulations (i.e., Ex.#1-5) prepared herein and commercially available lots of Skelaxin® 400-mg tablets. In dissolution Test-A, each 400-mg metaxalone tablet (Skelaxin® or experimental tablet) was placed into a glass peak vessel filled with 1000 mL of purified water which was maintained at room temperature (i.e., 25°C) and stirred at a paddle speed of 100 rpm. In dissolution Test-B, each 400-mg metaxalone tablet was placed into a standard glass vessel filled with 1000 mL of purified water which was maintained at 35°C and stirred at a paddle speed of 100 rpm. Finally, in dissolution Test-C, each 400-mg metaxalone tablet was placed into a glass peak vessel filled with 500 mL of a 0.1% w/v sodium lauryl sulfate (SLS) solution in water, which was maintained at 37°C and stirred at a paddle speed of 50 rpm. The metaxalone 30-minute drug dissolution rates (i.e., % drug dissolved within the first 30 minutes of dissolution) obtained from the five experimental metaxalone tablet formulations (Ex.41-5) and commercial lots of Skelaxin® 400-mg tablets using dissolution Test-A, Test-B and Test-C, are compared in Table 2.

**Table 2:** Metaxalone Drug Dissolution Rates (% drug dissolved in the first 30 minutes) obtained from experimental metaxalone tablet formulation (Ex. #1-5) and commercial Skelaxin® Tablets using 3 different dissolution tests (i.e., Test-A, Test-B and Test-C).

| METAXALONE TABLET FORMULATIONS | | | | | | |
|---|---|---|---|---|---|---|
| **Drug Dissolution Rate** (% Drug Dissolved in the first 30 minutes of dissolution) | **Ex. # 1** (BB5800040) 400-mg Tablets | **Ex. # 2** (BB5800087) 400-mg Tablets | **Ex.# 3** (BB5800047) 400-mg Tablets | **Ex. # 4** (BB5800056) 400-mg Tablets | **Ex. # 5** (NB1190: 48) 400-mg Tablets | **SKELAXIN®** (Com. Lots) 400-mg Tablets |
| **Dissolution Test-A** Peak Vessels - 1000 mL Water (25°C) - 100 rpm Paddles | 9% | 29% | 34% | 39% | 46% | 13% |
| **Dissolution Test-B** Std Vessels - 1000 mL Water (35°C)- 100 rpm Paddles | 22% | 40% | 48% | 52% | 57% | 28% |
| **Dissolution Test-C** Peak Vessels-500 mL 0.1% SLS (37°C)-50 rpm Paddles | 13% | 30% | 37% | 42% | 48% | 27% |

**Bioavailability Studies:** Five relative bioavailability (bioequivalence) studies were conducted under fasting (four studies) or non-fasting (one study) dosing conditions using each of the four experimental metaxalone tablets formulations, namely, Ex.#1-4, manufactured by Mutual Pharmaceutical Company, Inc. as the Test product and commercially available Skelaxin® 400-mg tablets manufactured by Mallinckrodt, Inc. for Carnrick Laboratories, Inc. (Division of Élan Pharmaceuticals) as the Reference product. Specifically, Ex.#1 (Lot # BB5800040) metaxalone 400-mg tablets were tested against Lot # GS639A of Skelaxin® 400-mg tablets under fasting dosing conditions on 35 healthy male volunteers. Ex.#2 (Lot # BB5800087) metaxalone 400-mg tablets were tested against Lot # GS1109A of Skelaxin® 400-mg tablets under fasting and non-fasting conditions on 43 and 24 healthy male volunteers, respectively. Ex.#3 (Lot # BB5800047) metaxalone 400-mg tablets were tested against Lot # GS639A of Skelaxin® 400-mg tablets under fasting conditions on 24 healthy male volunteers. Finally, Ex.#4 (Lot # BB5800056) metaxalone 400-mg tablets were tested against Lot # GS803A of Skelaxin® 400-mg tablets under fasting dosing conditions on 8 healthy male volunteers. In each study, a randomized, two-way crossover design was used to compare the relative bioavailability (rate and extent of drug absorption) of the Test (A) and Reference (B) products. The human subjects participating in each study were initially randomly assigned a sequence AB or BA. A single oral dose of the Test (A) and Reference (B) product was administered to the volunteers on two separate occasions under fasting or, in one study involving Ex.#2, non-fasting conditions, with at least a 7-day washout period between doses. Food and fluid intake were controlled during each confinement period.

Depending on the biostudy, about 18 to 23 blood samples per subject were collected each period at prescheduled time intervals spanning from time 0 hours (obtained within one hour prior to dose administration) to 36 or 48 hours for drug content analysis. Slight deviations from some scheduled times of sample collection were acceptable and, in such case, the actual time intervals were used in the statistical analysis. After analytical determination of the metaxalone plasma concentrations in each sample by the Analytical Laboratory of PRACS Institute, Ltd., the results were evaluated by the Statistical Division of PRACS Institute, Ltd. in order to calculate the pharmacokinetic parameters using WinNonlin™, Version 3.1, software designed specifically for analyzing pharmacokinetic data. WinNonlin™ Model 200 for extravascular input was utilized. All other computations were completed using SAS®, Version 8.2 software for Windows.

The following pharmacokinetic parameters were computed from the plasma concentration data using the actual sample collection times:

$AUC_{0-t}$     Area under the plasma concentration-time curve (ng-hr/mL) from time zero to the time of the last quantifiable concentration (t), calculated using the linear trapezoidal rule:

$$\sum_i (t_i - t_{i-1})(C_i + C_{i-1})/2, \quad i = 1 \text{ to } t,$$

where $C_i$ is the plasma concentration at time ti.

$AUC_{inf}$     Area under the plasma concentration curve from time zero extrapolated to infinity (ng-hr/mL), calculated by $AUC_{0-1} + (C_{last}/k_{elim})$, where $C_{last}$ is the last quantifiable concentration and $k_{elim}$ is the terminal elimination rate constant.

$C_{max}$     Maximum or peak concentration, obtained by inspection (ng/mL).

$T_{max}$     Time of maximum or peak concentration, obtained by inspection (hr).

$k_{elim}$     Terminal elimination rate constant (1/hr). This value was estimated by linear regression on the terminal phase of the semi-logarithmic concentration versus time curve.

$t_{1/2}$     Half life of the product (hr), calculated by $\ln(2)/k_{elim}$,

Natural logarithmic (ln) transformations were computed for $C_{max}$, $AUC_{0-t}$ and $AUC_{inf}$. In this way, the geometric means of the ln-transforined Least Squares Means were calculated.

In each study, an analysis of variance (ANOVA) was performed on each of the pharmacokinetic parameters using SAS® software. The ANOVA model containing factors for sequence of products, subjects within sequence, periods and products was utilized in comparing the effects between the Test and Reference products. Differences were declared statistically significant at the 5% level. A 90% confidence interval about the ratio of the mean test value to mean reference value was calculated for all of the pharmacokinetic parameters. The power of the ANOVA to detect a difference equal to 20% of the reference mean was also calculated with the SAS® software. The calculations for the power and confidence interval used the least squares means (LSMEANS) and the standard error of the estimate, both generated by the SAS® software. The ratio of the geometric means for the ln-transformed data and the corresponding 90% confidence intervals were calculated for $AUC_{0-t}$, $AUC_{inf}$, and $C_{max}$, as well. It should be noted that the lower limit of quantitation for metaxalone was 10.00 ng/mL. For statistical analysis, subject sample values below the lower limit of quantitation (BLQ) were reported as zero.

Results of non-transformed and ln-transformed computations of the critical pharmacokinetic parameters and their corresponding Test/Reference ratio percents obtained from each study are given in Tables 3 and 4, respectively, where it can be seen that the Test metaxalone tablets are "less-bioavailable" (i.e., Ex.#1), "bioequivalent" (i.e., Ex.#2, at fasting and non-fasting dosing conditions), "more-bioavailable" (i.e., Ex.#3), or "significantly-more-bioavailable" (i.e., Ex.#4) than the Reference Skelaxin® 400-mg tablets. The statistical analysis summary Tables related to the fasting and non-fasting bioequivalence studies conducted using Ex.#2 metaxalone 400-mg tablets (Lot # BB5800087) as the Test product and Skelaxin® 400-mg tablets (Lot # GS1109A) as the Reference product are shown in Figures 1 and 2. Furthermore, the absorption profiles (i.e., curves of the mean plasma concentrations of metaxalone obtained from all subjects in each time interval against time) after single oral administration of experimental metaxalone tablets Ex.#2 (both normal and semilogarithmic plots) or Ex.#4 (only normal plot) dosed as the Test products against the Reference Skelaxin® tablets are compared in Figures 3, 4 and 5.

**Table 3:** Comparison of oral bioavailability of metaxalone as defined by the values of the non-transformed Least Squares Means of critical pharmacokinetic parameters and the non-transformed Mean Test/Reference ratio percents of $C_{max}$, $AUC_{inf}$, $AUC_{0-t}$, $T_{max}$, $k_{elim}$ and $t_{1/2}$ obtained from clinical biostudies on healthy human volunteers in two period crossover studies involving a single dose oral administration under fasting conditions of each of the four experimental metaxalone tablet formulations (Ex.#1-4) used as the Test product versus Skelaxin® 400-mg tablets used as the Reference product. The projected values expected to be obtained from a similar bioequivalence study comparing the fifth experimental tablet formulation (Ex.#5) against the Reference Skelaxin® 400-mg tablets are also tabulated.

### METAXALONE TABLET FORMULATIONS Tested Against SKELAXIN 400-mg Tablets

**Non-Transformed Data:**

| Non-transformed Least Squares Means of Critical PK Parameters and Test/ Reference Ratios | Ex. # 1 | Ex. # 2 | Ex. # 3 | Ex. # 4 | Ex. # 5 |
|---|---|---|---|---|---|
| Means of Non-Transformed Least Squares Means and Ratio Percents (Test/ Skelaxin-Reference) of $C_{max}$, $AUC_{inf}$ and $AUC_{0-t}$ and $T_{max}$ | (BB5800040) *Less Bioavailable Than Skelaxin* | (BB5800087) *Bioequivalent To Skelaxin* | (BB5900047) *More Bioavailable Than Skelaxin* | (BB5800056) *Significantly More Bioavailable Than Skelaxin Reference* <u>*Maximally Bioavailable Liquisolid Product*</u> | (NB1190:48) <u>*Projected to be*</u> *Significantly More Bioavailable Than Skelaxin Reference* <u>*Maximally Bioavailable Liquisolid Product*</u> |
| $C_{max}$ **Means** (Test & Ref): | (in ng/mL) | (in ng/mL) | (in ng/mL) | (in ng/mL) | (in ng/mL) |
| $C_{max}$ **of Test Product** | **518** | **710** | **1,796** | **3,022** | **3,109** |
| $C_{max}$ **of Skelaxin Reference** | *669* | *672* | *777* | *932* | *713* |
| (N = number of subjects-Fasting) | (N = 35) | (N = 43) | (N = 24) | (N = 8) | *(Projected Values)* |
| $C_{max}$ **Ratio %** *(Fasting)* | **77.4%** | **105.6%** | **231.2%** | **324.3%** | **436.0%** |
| (N = number of subjects-Fasting) | (N = 35) | (N = 43) | (N = 24) | (N = 8) | *(Projected Value base on Test-C dissolution data)* |
| {*Lower-Upper 90% C.I. Limits*} | *{61.8% - 93.0%}* | *{94.9%-116.4%}* | *{206% - 257%}* | *{281% - 368%}* | |
| $AUC_{inf}$ **Means** (Test & Ref): | (in ng-hr/mL) | (in ng-hr/mL) | (in ng-hr/mL) | (in ng-hr/mL) | (in ng-hr/mL) |
| $AUC_{inf}$ **of Test Product** | **4,569** | **4,990** | **8,223** | **11,130** | **12,664** |
| $AUC_{inf}$ **of Skelaxin Reference** | *5,215* | *5,633* | *5,956* | *6,260* | *5,616* |
| (N = number of subjects-Fasting) | (N = 35) | (N = 43) | (N = 24) | (N = 8) | *(Projected Values)* |
| $AUC_{inf}$ **Ratio %** *(Fasting)* | **87.6%** | **88.6%** | **138.1%** | **177.8%** | **225.5%** |
| (N = number of subjects) | (N = 35) | (N = 43) | (N = 24) | (N = 8) | *(Projected Value based on Test-C dissolution data)* |
| {*Lower-Upper 90% C.I. Limits*} | *{79.4% - 95.8%}* | *{82.6% - 94.6%}* | *{124% - 154%}* | *{161% - 195%}* | |

(continued)

**METAXALONE TABLET FORMULATIONS Tested Against SKELAXIN 400-mg Tablets**

**Non-Transformed Data:**

| Non-transformed Least Squares Means of Critical PK Parameters and Test/ Reference Ratios | Ex. # 1 | Ex. # 2 | Ex. # 3 | Ex. # 4 | Ex. # 5 |
|---|---|---|---|---|---|
| Means of Non-Transformed Least Squares Means and Ratio Percents (Test/ Skelaxin-Reference) of $C_{max}$, $AUC_{inf}$ and $AUC_{0-t}$ and $T_{max}$ | (BB5800040) *Less Bioavailable Than Skelaxin* | (BB5800087) *Bioequivalent To Skelaxin* | (BB5900047) *More Bioavailable Than Skelaxin* | (BB5800056) *Significantly More Bioavailable Than Skelaxin Reference Maximally Bioavailable Liquisolid Product* | (NB1190:48) <u>*Projected to be*</u> *Significantly More Bioavailable Than Skelaxin Reference* <u>*Maximally Bioavailable Liquisolid Product*</u> |
| $AUC_{0-t}$ **Means** (Test & Ref): | (in ng-hr/mL) | (in ng-hr/mL) | (in ng-hr/mL) | (in ng-hr/mL) | (in ng-hr/mL) |
| $AUC_{0-t}$ **of Test Product** | **4,365** | **4,728** | **8,138** | **11,064** | **12,590** |
| $AUC_{0-t}$ **of Skelaxin Reference** | *5,074* | *5,381* | *5,672* | *6,000* | *5,392* |
| (N = number of subjects-Fasting) | (N = 35) | (N = 43) | (N = 24) | (N = 8) | *(Projected Values)* |
| $AUC_{0-t}$ **Ratio %** *(Fasting)* | **86.0%** | **87.9%** | **143.5%** | **184.4%** | **233.5%** |
| (N = number of subjects) | (N=35) | (N=43) | (N=24) | (N = 8) | *(Projected Value based on Tavi-C dissolution data)* |
| {*Lower-Upper 90% C.I. Limits*} | {*77.9% - 94.2%*} | {*81.7% - 94.1%*} | {*129% - 158%*} | {*167% - 202%*} | |
| $T_{max}$ **Means** (Test & Ref.): | (in hours) | (in hours) | (in hours) | (in hours) | |
| $T_{max}$ **of Test Product** | **3.69** | **3.50** | **3.02** | **2.94** | *N/A* |
| $T_{max}$ **of Skelaxin Reference** | *3.43* | *4.07* | *3.40* | *3.94* | |
| (N = number of subjects-Fasting) | (N=35) | (N=43) | (N=24) | (N = 8) | |
| $T_{max}$ **Ratio %** *(Fasting)* | **107.6%** | **86.1%** | **88.8%** | **74.5%** | |
| (N=numberofsubjects) | (N=35) | (N=43) | (N = 24) | (N=8) | *N/A* |
| *(Lower-Upper 90% G.L Limits)* | {*91.8% - 124%*} | {*70.1% - 102.8%*} | {*76% - 102%*} | {*54%-95%*} | |
| $k_{elim}$ **Means** (Test & Ref.): | (in hrs$^{-1}$) | (in hrs$^{-1}$) | (in hrs$^{-1}$) | (in hrs$^{-1}$) | |
| $k_{elim}$ **of Test Product** | **0.1116** | **0.1178** | **0.3794** | **0.4413** | *N/A* |
| $k_{elim}$ **of Skelaxin Reference** | *0.1157* | *0.1324* | *0.1081* | *0.1103* | |
| (N = number of subjects-Fasting) | (N=35) | (N = 43) | (N=24) | (N = 8) | |
| $k_{elim}$ **Ratio %** *(Fasting)* | **96.5%** | **89.0%** | **351.0%** | **398.2%** | |
| (N = number of subjects) | (N = 35) | (N=43) | (N = 24) | (N = 8) | *N/A* |

(continued)

**METAXALONE TABLET FORMULATIONS Tested Against SKELAXIN 400-mg Tablets**

**Non-Transformed Data:**

| Non-transformed Least Squares Means of Critical PK Parameters and Test/ Reference Ratios | Ex. # 1 | Ex. # 2 | Ex. # 3 | Ex. # 4 | Ex. # 5 |
|---|---|---|---|---|---|
| Means of Non-Transformed Least Squares Means and Ratio Percents (Test/Skelaxin-Reference) of $C_{max}$, $AUC_{inf}$ and $AUC_{0-t}$ and $T_{max}$ | (BB5800040) *Less Bioavailable Than Skelaxin* | (BB5800087) *Bioequivalent To Skelaxin* | (BB5900047) *More Bioavailable Than Skelaxin* | (BB5800056) *Significantly More Bioavailable Than Skelaxin Reference* <u>*Maximally Bioavailable Liquisolid Product*</u> | (NB1190:48) <u>*Projected to be*</u> *Significantly More Bioavailable Than Skelaxin Reference* <u>*Maximally Bioavailable Liquisolid Product*</u> |
| *{Lower-Upper 90% C.I. Limits}* | *{84.6% - 108%}* | *{74.3% - 103.6%}* | *{296% - 407%}* | *{338% - 458%}* | |
| **$t_{1/2}$ Means** (Test & Ref.): | (in hours) | (in hours) | (in hours) | (in hours) | |
| **$t_{1/2}$ of Test Product** | **7.75** | **7.58** | **2.46** | **1.64** | *N/A* |
| **$t_{1/2}$ of Skelaxin Reference** | **6.66** | **6.13** | **7.66** | **6.75** | |
| (N = number of subjects-Fasting) | (N = 35) | (N = 43) | (N=24) | (N = 8) | |
| **$t_{1/2}$ Ratio %** *(Fasting)* | **116.4%** | **123.5%** | **32.1%** | **24.3%** | |
| (N = number of subjects) | (N=35) | (N = 43) | (N = 24) | (N = 8) | *N/A* |
| *{Lower-Upper 90% C.I. Limits}* | *{87.7% - 145%}* | *{107% - 140%}* | *{14% - 51%}* | *{5% - 44%}* | |

**Table 4:** Comparison of oral bioavailability of metaxalone as defined by the geometric means of the ln-transformed least squares means of critical pharmacokinetic parameters and the ln-transformed geometric means of Test/Reference ratio percents of $C_{max}$, $AUC_{inf}$ and $AUC_{0-t}$, obtained from clinical biostudies on healthy human volunteers in two period crossover studies involving a single dose oral administration under fasting and non-fasting conditions of each of the four experimental metaxalone tablet formulations (Ex.#1-4) used as the Test product versus Skelaxin® 400-mg tablets used as the Reference product. The projected values expected to be obtained from a similar bioequivalence study comparing the fifth experimental tablet formulation (Ex.#5) against the Reference Skelaxin® 400-mg tablets are also tabulated.

**METAXALONE TABLET FORMULATIONS Tested Against SKELAXIN 400-mg** Tablets

**Ln-Transformed Data:**

| **Geometric Means of ln-transformed Least Squares Means of Critical PK Parameters and Test/ Reference Ratios** | **Ex. # 1** | **Ex. # 2** | **Ex. # 3** | **Ex. # 4** | **Ex. # 5** |
|---|---|---|---|---|---|
| Geometric Means of lntransformed Least Square Means and Ratio Percents (Test/Skelaxin) of $C_{max}$, $AUC_{inf}$ and $AUC_{0-t}$ | (BB5800040) *Less Bioavailable Than Skelaxin* | (BB5800087) *Bioequivalent To Skelaxin* | (BB5800047) *More Bioavailable Than Skelaxin* | (BB5800056) *Significantly More Bioavailable Than Skelaxin Reference* <u>*Maximally Bioavailable Liquisolid Product*</u> | (NB1190:48) <u>*Projected to be*</u> *Significantly More Bioavailable Than Skelaxin Reference* <u>*Maximally Bioavailable Liquisolid Product*</u> |
| $C_{max}$ **Geometric Means:** | (in ng/mL) | (in ng/mL) | (in ng/mL) | (in ng/mL) | (in ng/mL) |
| $C_{max}$ **of Test Product** | **425** | **647** | **1,669** | **2,936** | **3,141** |
| *$C_{max}$ of Skelaxin Referertce* | *620* | *606* | *721* | *851* | *653* |
| (N = number of subjects -Fasting) | (N = 35) | (N = 43) | (N=24) | (N = 8) | *(Projected Values)* |
| $C_{max}$ **Ratio %** *(Fasting)* | **68.5%** | **106.9%** | **231.6%** | **344.9%** | **480.9%** |
| (N = number of subjects) | (N = 35) | (N = 43) | (N = 24) | (N = 8) | *(Projected Value* |
| {*Lower-Upper 90% C.I Limits*} | {55.5% - 84.5%} | {95.8% -119.3%} | {202% - 266%} | {263% - 452%} | *based on Test-C dissolution data)* |
| $AUC_{inf}$ **Geometric Means:** | (in ng-hr/mL) | (in ng-hr/mL) | (in ng-hr/mL) | (in ng-hr/mL) | (in ng-hr/mL) |
| $AUC_{inf}$ **of Test Product** | **4,196** | **4,518** | **7,518** | **10,942** | **12,508** |

(continued)

| METAXALONE TABLET FORMULATIONS Tested Against SKELAXIN 400-mg Tablets | | | | | |
|---|---|---|---|---|---|
| **Ln-Transformed Data:** | | | | | |
| **Geometric Means of In-transformed Least Squares Means of Critical PK Parameters and Test/ Reference Ratios** | **Ex. # 1** | **Ex. # 2** | **Ex. # 3** | **Ex. # 4** | **Ex. # 5** |
| Geometric Means of Intransformed Least Square Means and Ratio Percents (Test/Skelaxin) of $C_{max}$, $AUC_{inf}$ and $AUC_{0-t}$ | (BB5800040) *Less Bioavailable Than Skelaxin* | (BB5800087) *Bioequivalent To Skelaxin* | (BB5800047) *More Bioavailable Than Skelaxin* | (BB5800056) *Significantly More Bioavailable Than Skelaxin Reference <u>Maximally Bioavailable Liquisolid Product</u>* | (NB1190:48) *<u>Projected to be</u> Significantly More Bioavailable Than Skelaxin Reference <u>Maximally Bioavailable Liquisolid Product</u>* |
| ***$AUC_{inf}$ of Skelaxin Reference*** | ***4,939*** | ***5,107*** | ***5,453*** | ***5,920*** | ***5,188*** |
| (N = number of subjects-Fasting) | (N = 35) | (N = 43) | (N = 24) | (N = 8) | *(Projected Values)* |
| **$AUC_{inf}$ Ratio % *(Fasting)*** | **85.0%** | **88.5%** | **137.9%** | **184.8%** | **241.1%** |
| (N = number of subjects) | (N = 35) | (N = 43) | (N=24) | (N = 8) | *(Projected Value* |
| *{Lower-Upper 90% C.I. Limits}* | *{77.2% - 93.4%}* | *{83.5% - 93.8%}* | *{124% - 159%}* | *{157% - 218%}* | *based on Test-C dissolution data)* |
| **$AUC_{0-t}$ Geometric Means:** | (in ng-hr/mL) | (in ng-hr/mL) | (in ng-hr/mL) | (in ng-hr/mL) | (in ng-hr/mL) |
| **$AUC_{0-t}$ of Test Product** | **3,932** | **4,267** | **7,428** | **10,872** | **12,400** |
| ***$AUC_{0-t}$ of Skelaxin Reference*** | ***4,784*** | ***4,875*** | ***5,162*** | ***5,661*** | ***4,966*** |
| (N = number of subjects -Fasting) | (N = 35) | (N = 43) | (N = 24) | (N = 8) | *(Projected Values)* |
| **$AUC_{0-t}$ Ratio % *(Fasting)*** | **85.0%** | **88.5%** | **143.9%** | **192.0%** | **249.7%** |
| (N = number of subjects) | (N=35) | (N=43) | (N=24) | (N=8) | *(Projected Value based on Test-C* |
| *{Lower-Upper 90% C.I Limits}* | *{77.2% - 93.4%}* | *{83.5% - 93.8%}* | *{129% - 161%}* | *{163% - 226%}* | *dissolution data)* |

(continued)

**METAXALONE TABLET FORMULATIONS Tested Against SKELAXIN 400-mg** Tablets

**Ln-Transformed Data:**

| **Geometric Means of In-transformed Least Squares Means of Critical PK Parameters and Test/ Reference Ratios** | **Ex. # 1** | **Ex. # 2** | **Ex. # 3** | **Ex. # 4** | **Ex. # 5** |
|---|---|---|---|---|---|
| Geometric Means of Intransformed Least Square Means and Ratio Percents (Test/Skelaxin) of $C_{max}$, $AUC_{inf}$ and $AUC_{0-t}$ | (BB5800040) *Less Bioavailable Than Skelaxin* | (BB5800087) *Bioequivalent To Skelaxin* | (BB5800047) *More Bioavailable Than Skelaxin* | (BB5800056) *Significantly More Bioavailable Than Skelaxin Reference <u>Maximally Bioavailable Liquisolid Product</u>* | (NB1190:48) *<u>Projected to be</u> Significantly More Bioavailable Than Skelaxin Reference <u>Maximally Bioavailable Liquisolid Product</u>* |
| **$C_{max}$ Ratio % *(Non-Fasting)*** | | **93.0%** | | | |
| (N = number of subjects) | N/A | (N = 24) | N/A | N/A | N/A |
| *{Lower-Upper 90% C.I. Limits}* | | *{74.7%. - 115.8%}* | | | |
| **$AUC_{int}$ Ratio % *(Non-Fasting)*** | | **86.9%** | | | |
| (N = number of subjects) | N/A | (N = 24) | N/A | N/A | N/A |
| *{Lower-Upper 90% C.I. Limits}* | | *{80.0% - 94.4%}* | | | |
| **$AUC_{0-t}$ Ratio % *(Non-Fasting)*** | | **90.1%** | | | |
| (N = number of subjects) | N/A | (N = 24) | N/A | N/A | N/A |
| *{Lower-Upper 90% C.I Limits}* | | *{82.1% - 98.9%}* | | | |

**Discussion of the Results:** As shown in Tables 2-4 above, Ex.#1 metaxalone tablet formulation (BB5800040) which demonstrated in all three dissolution tests slower drug dissolution rates than the Skelaxin® 400-mg tablets, was also found to be "less bioavailable" than the reference Skelaxin® product of NDA #13-217. Surprisingly, however, as shown in Figures 1-4 and Tables 2-4, even though Ex.#2 metaxalone tablet formulation (BB5800087) exhibited significantly faster drug dissolution rates than the reference Skelaxin® 400-mg tablets in Dissolution Test-A and Test-B, it was found to be "bioequivalent" to the Skelaxin® tablets of NDA #13-217. It is therefore possible for metaxalone tablets to be prepared which can display drug dissolution rates higher than those of their commercial Skelaxin® counterparts while at the same time demonstrating at both fasting and non-fasting conditions bioavailability properties similar to those of the reference Skelaxin® product of NDA #13-217. On the other hand, as shown in Tables 2-4, Ex.#3 metaxalone tablet formulation (BB5800047) which does not contain a nonvolatile liquid, demonstrated faster drug dissolution rates in all three dissolution tests than the reference Skelaxin® 400-mg tablets and was found to be "more bioavailable" than the reference Skelaxin® product of NDA #13-217.

Most importantly, however, Ex.#4 metaxalone liquisolid tablet formulation (BB5800056) which contains a non-volatile liquid (i.e., polyethylene glycol 400), not only demonstrated faster drug dissolution rates in all three dissolution tests than Ex.#3 metaxalone tablets and the reference Skelaxin® 400-mg tablets but it was also found to be significantly-more-bioavailable than the reference Skelaxin® product of NDA #13-217. Furthermore, Ex.#5 metaxalone liquisolid tablets (NB1190:48) which contain larger amounts of another nonvolatile liquid (i.e., polysorbate 80) demonstrated faster drug dissolution rates than even the maximally bioavailable Ex.#4 metaxalone liquisolid tablet formulation. Mathematical projections and modeling based on the drug dissolution rates obtained from dissolution Test-C lead to the conclusion that Ex.#5 metaxalone should be expected to display Test/Reference $C_{max}$ and $AUC_{inf}$ ratio percents which would be even greater than those obtained from the maximally bioavailable Ex.#4 metaxalone tablets. Such projected maximally enhanced bioavailability properties of Ex.#5 as compared to those of the reference Skelaxin® product of NDA #13-217 are included in Tables 3 and 4.

In summary, based on the results presented herein, it is apparent that pharmaceutically acceptable solid dosage forms of an essentially water-insoluble drug such as metaxalone can be prepared using the ingredient compositions and manufacturing methods discussed herein. Such compositions demonstrate improved drug dissolution rates as compared to commercial lots of the essentially water-insoluble drug such as Skelaxin® 400-mg tablets, while at the same time being bioequivalent to, more bioavailable than, or significantly more bioavailable than conventionally made and/or commercially available reference products such as the Skelaxin® tablets of NDA #13-217.

As shown herein, metaxalone products significantly more bioavailable than the reference Skelaxin® tablets of NDA #13-217 can be prepared to contain various amounts of nonvolatile liquids using Liquisolid technology. Based on the methods, compositions, results and projections presented herein, it is apparent that such metaxalone Liquisolid tablets are maximally bioavailable thereby leading to new metaxalone dosage forms which, even if they contain smaller doses of metaxalone per unit dose as compared to the reference Skelaxin® 400-mg tablets of NDA #13-217 (say liquisolid metaxalone 50 mg to 350 mg tablets), they would still be bioequivalent to said Skelaxin® 400-mg tablets of NDA #13-217. Such maximally bioavailable low dose liquisolid metaxalone products represent unique, novel and commercially desirable products and are incorporated herein as one of the preferred embodiments of the present invention. Finally, it should be also expected that metaxalone, products prepared according to the present invention and demonstrating maximally enhanced bioavailability properties under fasting conditions as compared to the Skelaxin® tablets, would not present any food effects on oral bioavailability, i.e., they should demonstrate similar oral bioavailability under both fasting and non-fasting dosing conditions.

As used in this specification and the appended claims, the singular forms "a," "an" and "the" include plural references unless the content clearly dictates otherwise. Thus, for example, reference to "an excipient" includes a mixture of two or more excipients.

Pharmaceutical solid dosage forms are described herein comprising an effective amount of metaxalone and at least one inactive powder excipient wherein said dosage form presents improved drug dissolution rate as compared to the metaxalone product of NDA #13-217. Preferably, the pharmaceutical solid dosage form contains at least one nonvolatile liquid. Preferably, the pharmaceutical solid dosage form is bioequivalent to the metaxalone product ofNDA #13-217 after oral administration to fasting or non-fasting human subjects. Preferably, the pharmaceutical solid dosage form is more bioavailable than the metaxalone product of NDA #13-217 after oral administration to fasting or non-fasting human subjects. Preferably, the pharmaceutical solid dosage form is significantly more bioavailable than the metaxalone product of NDA #13-217 after oral administration to fasting or non-fasting human subjects.

Pharmaceutical solid dosage forms are described herein comprising metaxalone and at least one inactive powder excipient and wherein: (i) 13% by weight or greater of said metaxalone is dissolved about 30 minutes after said dosage form is placed in a peak glass dissolution vessel filled with 1000 mL of purified water, maintained at 25°C and stirred at a paddle speed of 100 rpm using a USP Type II (paddle) apparatus; or (ii) 28% by weight or greater of said metaxalone is dissolved about 30 minutes after said dosage form is placed in a standard glass dissolution vessel filled with 1000 mL of purified water, maintained at 35°C and stirred at a paddle speed of 100 rpm using a USP Type II (paddle) apparatus; or (iii) 27% by weight or greater of said metaxalone is dissolved about 30 minutes after said dosage form is placed in a peak glass dissolution vessel filled with 500 mL of an aqueous solution of 0.1 % by weight of Sodium Lauryl Sulfate per volume of water, maintained at 37°C and stirred at a paddle speed of 50 rpm using a USP Type II (paddle) apparatus.

Preferably, the pharmaceutical solid dosage form comprises (i) 29% by weight or greater of said metaxalone is dissolved about 30 minutes after said dosage form is placed in a peak glass dissolution vessel filled with 1000 mL of purified water, maintained at 25°C and stirred at a paddle speed of 100 rpm using a USP Type II (paddle) apparatus; or (ii) 40% by weight or greater of said metaxalone is dissolved about 30 minutes after said dosage form is placed in a standard glass dissolution vessel filled with 1000 mL of purified water, maintained at 35°C and stirred at a paddle speed of 100 rpm using a USP Type II (paddle) apparatus; or (iii) 30% by weight or greater of said metaxalone is dissolved about 30 minutes after said dosage form is placed in a peak glass dissolution vessel filled with 500 mL of an aqueous solution of 0.1 % by weight of Sodium Lauryl Sulfate per volume of water, maintained at 37°C and stirred at a paddle speed of 50 rpm using a USP Type II (paddle) apparatus.

More preferably, the pharmaceutical solid dosage form comprises (i) 34% by weight or greater of said metaxalone is dissolved about 30 minutes after said dosage form is placed in a peak glass dissolution vessel filled with 1000 mL of purified water, maintained at 25 °C and stirred at a paddle speed of 100 rpm using a USP Type II (paddle) apparatus; or (ii) 48% by weight or greater of said metaxalone is dissolved about 30 minutes after said dosage form is placed in a standard glass dissolution vessel filled with 1000 mL of purified water, maintained at 35 °C and stirred at a paddle speed of 100 rpm using a USP Type II (paddle) apparatus; or (iii) 37% by weight or greater of said metaxalone is dissolved about 30 minutes after said dosage form is placed in a peak glass dissolution vessel filled with 500 mL of an aqueous solution of 0.1 % by weight of Sodium Lauryl Sulfate per volume of water, maintained at 37 °C and stirred at a paddle speed of 50 rpm using a USP Type II (paddle) apparatus.

Even more preferably, the pharmaceutical solid dosage form comprises (i) 39% by weight or greater of said metaxalone is dissolved about 30 minutes after said dosage form is placed in a peak glass dissolution vessel filled with 1000 mL of purified water, maintained at 25 °C and stirred at a paddle speed of 100 rpm using a USP Type II (paddle) apparatus; or (ii) 52% by weight or greater of said metaxalone is dissolved about 30 minutes after said dosage form is placed in a standard glass dissolution vessel filled with 1000 mL of purified water, maintained at 35 °C and stirred at a paddle speed of 100 rpm using a USP Type II (paddle) apparatus; or (iii) 42% by weight or greater of said metaxalone is dissolved about 30 minutes after said dosage form is placed in a peak glass dissolution vessel filled with 500 mL of an aqueous solution of 0.1 % by weight of Sodium Lauryl Sulfate per volume of water, maintained at 37 °C and stirred at a paddle speed of 50 rpm using a USP Type II (paddle) apparatus.

The form may comprise (i) 46% by weight or greater of said metaxalone is dissolved about 30 minutes after said dosage form is placed in a peak glass dissolution vessel filled with 1000 mL of purified water, maintained at 25 °C and stirred at a paddle speed of 100 rpm using a USP Type II (paddle) apparatus; or (ii) 57% by weight or greater of said metaxalone is dissolved about 30 minutes after said dosage form is placed in a standard glass dissolution vessel filled with 1000 mL of purified water, maintained at 35 °C and stirred at a paddle speed of 100 rpm using a USP Type II (paddle) apparatus; or (iii) 48% by weight or greater of said metaxalone is dissolved about 30 minutes after said dosage form is placed in a peak glass dissolution vessel filled with 500 mL of an aqueous solution of 0.1 % by weight of Sodium Lauryl Sulfate per volume of water, maintained at 37 °C and stirred at a paddle speed of 50 rpm using a USP type II (paddle) apparatus.

The form may contain at least one nonvolatile liquid. The dosage form may be bioequivalent to the metaxalone product of NDA # 13-217 after oral administration to fasting or non-fasting human subjects. The dosage form may be more bioavailable than the metaxalone product of NDA # 13-217 after oral administration to fasting or non-fasting human subjects. The dosage form may be significantly more bioavailable than the metaxalone product of NDA# 13-217 after oral administration to fasting or non-fasting human subjects.

Pharmaceutical solid dosage forms are described herein comprising metaxalone and at least one inactive powder excipient wherein said dosage form is bioequivalent to the metaxalone product of NDA #13-217 after oral administration to fasting or non-fasting human subjects, and wherein: (i) more than 13% by weight and less than 39% by weight of said metaxalone is dissolved about 30 minutes after said dosage form is placed in a peak glass dissolution vessel filled with 1000 mL of purified water, maintained at 25°C and stirred at a paddle speed of 100 rpm using a USP Type II (paddle) apparatus; or (ii) more than 28% by weight and less than 52% by weight of said metaxalone is dissolved about 30 minutes after said dosage form is placed in a standard glass dissolution vessel filled with 1000 mL of purified water, maintained at 35°C and stirred at a paddle speed of 100 rpm using a USP Type II (paddle) apparatus; or (iii) more than 27% by weight and less than 42% by weight of said metaxalone is dissolved about 30 minutes after said dosage form is placed in a peak glass dissolution vessel filled with 500 mL of an aqueous solution of 0.1 % by weight of Sodium Lauryl Sulfate per volume of water, maintained at 37°C and stirred at a paddle speed of 50 rpm using a USP Type II (paddle) apparatus.

The dosage form may contain at least one non-volatile liquid. The dosage form may be more bioavailable than the metaxalone product of NDA #13-217 after oral administration to fasting or non-fasting human subjects. The dosage form may be significantly more bioavailable than the metaxalone product of NDA #13-217 after oral administration to fasting or non-fasting human subjects.

Pharmaceutical solid dosage forms are described herein comprising metaxalone, at least one inactive powder excipient and at least one non-volatile solvent, wherein said dosage form is more bioavailable than the metaxalone product of NDA#13-217 and wherein: (i) 39% by weight or greater of said metaxalone is dissolved about 30 minutes after said dosage form is placed in a peak glass dissolution vessel filled with 1000 mL of purified water, maintained at 25°C and stirred at a paddle speed of 100 rpm using a USP Type II (paddle) apparatus; or (ii) 52% by weight or greater of said metaxalone is dissolved about 30 minutes after said dosage form is placed in a standard glass dissolution vessel filled with 1000 mL of purified water, maintained at 35°C and stirred at a paddle speed of 1 00 rpm using a USP Type II (paddle) apparatus; or (iii) 42% by weight or greater of said metaxalone is dissolved about 30 minutes after said dosage form is placed in a peak glass dissolution vessel filled with 500 mL of an aqueous solution of 0.1 % by weight of Sodium Lauryl Sulfate per volume of water, maintained at 37°C and stirred at a paddle speed of 50 rpm using a USP Type II (paddle) apparatus.

The dosage form may be significantly more bioavailable than the metaxalone product of NDA#13-217 after oral administration to fasting or non-fasting human subjects. The solid dosage may contain at least one nonvolatile liquid.

Pharmaceutical solid dosage forms are described herein comprising metaxalone and at least one inactive powder excipient, wherein said dosage form is bioequivalent to the metaxalone product of NDA #13-217 after oral administration to fasting or non-fasting human subjects and wherein said dosage form contains a lower quantity of metaxalone as compared to the quantity of drug contained in each unit of the metaxalone products of NDA #13-217.

Pharmaceutical solid dosage forms are described herein comprising metaxalone and at least one inactive powder excipient, wherein said dosage form demonstrates similar oral bioavailability properties when dosed to fasting and to non-fasting human subjects.

A method of treating a musculoskeletal condition is described comprising administering a solid dosage form comprising an effective amount of metaxalone and at least one inactive powder excipient, wherein said administered dosage form demonstrates improved drug dissolution rates as compared to the metaxalone product ofNDA #13-217.

A method of treating a musculoskeletal condition is described comprising administering a solid dosage form comprising an effective amount of metaxalone, at least one inactive powder excipient and at least one on-volatile liquid, wherein said administered dosage form demonstrates improved drug dissolution rates as compared to the metaxalone product of NDA#13-217.

The method may comprise identifying a mammal suffering from said musculoskeletal condition. The method may comprise assessing a degree of discomfort associated with said musculoskeletal condition in a mammal. The method may involve said assessment being made before administering said dosage form. The method may involve said assessment being made after administering said dosage form.

Main Request

**Claims**

1. A pharmaceutical solid dosage form comprising metaxalone, at least one powder excipient selected from a group comprising sparingly water-soluble or essentially water-insoluble, natural or synthetic hydrocolloids and polymers, and at least one nonvolatile liquid,
prepared by a method comprising the steps of;
providing an effective amount of metaxalone;
providing at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;
dry-mixing the effective amount of metaxalone and the at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;
wetting the metaxalone/powder blend with the at least one nonvolatile liquid and at least one volatile liquid to form a mix;
granulating the mix to form a granulation;
drying and milling the granulation;
mixing with at least one other inactive powder excipient
and compressing into tablets to produce the pharmaceutical solid dosage form;
wherein the at least one nonvolatile liquid is selected from the group consisting of propylene glycol, glycerin, liquid polyethylene glycols, semisolid polyethylene glycols, pharmasolve, liquid polysorbates, semisolid polysorbates, liquid spans, semisolid spans, liquid cremophors, semisolid cremophors, liquid pluronics, semisolid pluronics, liquid myglyols, semisolid myglyols, nonvolatile oils, vitamin-E, lecithin, or combinations thereof;
the at least one volatile liquid is selected from the group consisting of methanol, ethanol, acetone, water, or combinations thereof;
and wherein:

(i) 29% by weight or greater of said metaxolone is dissolved about 30 minutes after said dosage form is placed In a peak glass dissolution vessel filled with 1000 mL of purified water, maintained at 25°C and stirred at a paddle speed of 100 rpm using a USP Type II (paddle) apparatus; or
(ii) 40% by weight or greater of said metaxalone is dissolved about 30 minutes after said dosage form is placed in a standard glass dissolution vessel filled with 1000 mL of purified water, maintained at 35°C and stirred at a paddle speed of 100 rpm using a USP Type II (paddle) apparatus; or

(iii) 30% by weight or greater of said metaxalone is dissolved about 30 minutes after said dosage form is placed in a peak glass dissolution vessel filled with 500 mL of an aqueous solution of 0.1% by weight of Sodium Lauryl Sulfate per volume of water, maintained at 37°C and stirred at a paddle speed of 50 rpm using a USP Type II (paddle) apparatus.

2. The pharmaceutical solid dosage form according to Claim 1 wherein:

(i) 34% by weight or greater of said metaxalone is dissolved about 30 minutes after said dosage form is placed in a peak glass dissolution vessel filled with 1000 mL of purified water, maintained at 25°C and stirred at a paddle speed of 100 rpm using a USP Type II (paddle) apparatus; or

(ii) 48% by weight or greater of said metaxalone is dissolved about 30 minutes after said dosage form is placed in a standard glass dissolution vessel filled with 1000 mL of purified water, maintained at 35°C and stirred at a paddle speed of 100 rpm using a USP Type II (paddle) apparatus; or

(iii) 37% by weight or greater of said metaxalone is dissolved about 30 minutes after said dosage form is placed in a peak glass dissolution vessel filled with 500 mL of an aqueous solution of 0.1 % by weight of Sodium Lauryl Sulfate per volume of water, maintained at 37°C and stirred at a paddle speed of 50 rpm using a USP Type II (paddle) apparatus.

3. The pharmaceutical solid dosage form according to Claim 1 wherein:

(i) 39% by weight or greater of said metaxalone is dissolved about 30 minutes after said dosage form is placed in a peak glass dissolution vessel filled with 1000 mL of purified water, maintained at 25°C and stirred at a paddle speed of 100 rpm using a USP Type II (paddle) apparatus; or

(ii) 52% by weight or greater of said metaxalone is dissolved about 30 minutes after said dosage form is placed in a standard glass dissolution vessel filled with 1000 mL of purified water, maintained at 35°C and stirred at a paddle speed of 100 rpm using a USP Type II (paddle) apparatus; or

(iii) 42% by weight or greater of said metaxalone is dissolved about 30 minutes after said dosage form is placed in a peak glass dissolution vessel filled with 500 mL of an aqueous solution of 0.1 % by weight of Sodium Lauryl Sulfate per volume of water, maintained at 37°C and stirred at a paddle speed of 50 rpm using a USP Type II (paddle) apparatus.

4. The pharmaceutical solid dosage form according to Claim 1 wherein:

(i) 46% by weight or greater of said metaxalone is dissolved about 30 minutes after said dosage form is placed in a peak glass dissolution vessel filled with 1000 mL of purified water, maintained at 250C and stirred at a paddle speed of 100 rpm using a USP Type II (paddle) apparatus; or

(ii) 57% by weight or greater of said metaxalone is dissolved about 30 minutes after said dosage form is placed in a standard glass dissolution vessel filled with 1000 mL of purified water, maintained at 35°C and stirred at a paddle speed of 100 rpm using a USP Type II (paddle) apparatus; or

(iii) 48% by weight or greater of said metaxalone is dissolved about 30 minutes after said dosage form is placed in a peak glass dissolution vessel filled with 500 mL of an aqueous solution of 0.1 % by weight of Sodium Lauryl Sulfate per volume of water, maintained at 37°C and stirred at a paddle speed of 50 rpm using a USP Type II (paddle) apparatus.

5. The pharmaceutical solid dosage form according to any preceding Claim wherein the dosage form is bioequivalent to or more bioavailable than the metaxalone product of NDA #13-217 after oral administration to fasting or non-fasting human subjects.

6. The pharmaceutical solid dosage form according to any of Claims 1 to 4 wherein the dosage form is more bioavailable than the metaxalone product of NDA #13-217 after oral administration to fasting or non-fasting human subjects.

7. The pharmaceutical solid dosage form according to any preceding Claim wherein the dosage form demonstrates improved drug dissolution rates as compared to the metaxalone product of NDA #13-217, wherein dissolution rates are measured 30 minutes after the pharmaceutical solid dosage form or the metaxalone product of NDA#13-217 is placed in

(i) a peak glass dissolution vessel filled with 1000 mL of purified water, maintained at 25°C and stirred at a paddle speed of 100 rpm using a USP Type II (paddle) apparatus; or

(ii) a standard glass dissolution vessel filled with 1000 mL of purified water, maintained at 35°C and stirred at a paddle speed of 100 rpm using a USP Type II (paddle) apparatus; or

(iii) a peak glass dissolution vessel filled with 500 mL of an aqueous solution of 0.1% by weight of Sodium Lauryl Sulfate per volume of water, maintained at 37°C and stirred at a paddle speed of 50 rpm using a USP Type II (paddle) apparatus.

8. A pharmaceutical solid dosage form according to any preceding Claim for use in the treatment of a musculoskeletal condition.

9. A method of making a pharmaceutical solid dosage form according to any one of the preceding claims comprising:

providing an effective amount of metaxalone;

providing at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;

dry-mixing the effective amount of metaxalone and the at least one powder excipient selected from the group consisting of sodium alginate, ammonium alginate, sodium carboxymethyl cellulose, microcrystalline cellulose, pregelatinized starch, sodium starch glycolate, methacrylic acid copolymers, or combinations thereof;

wetting the metaxalone/powder blend with at least one nonvolatile liquid and at least one volatile liquid to form a mix;

granulating the mix to form a granulation;

drying and milling the granulation;

mixing with at least one other powder excipient;

and compressing into tablets to produce the pharmaceutical solid dosage form;

wherein the at least one nonvolatile liquid is selected from the group consisting of propylene glycol, glycerin, liquid polyethylene glycols, semisolid polyethylene glycols, pharmasolve, liquid polysorbates, semisolid polysorbates, liquid spans, semisolid spans, liquid cremophors, semisolid cremophors, liquid pluronics, semisolid pluronics, liquid myglyols, semisolid myglyols, nonvolatile oils, vitamin-E, lecithin, or combinations thereof; and the at least one volatile liquid is selected from the group consisting of methanol, ethanol, acetone, water, or combinations thereof.

10. A method of making a pharmaceutical solid dosage form for treating a musculoskeletal condition of any of the preceding claims wherein said dosage form is bioequivalent to or more bioavailable than the metaxalone product of NDA #13-217 after oral administration to fasting or non-fasting human subjects.

11. A method of making a pharmaceutical solid dosage form for treating a musculoskeletal condition of any of the preceding claims wherein said dosage form is more bioavailable than the metaxalone product of NDA #13-217 after oral administration to fasting or non-fasting human subjects.

12. A method of making a pharmaceutical solid dosage form for treating a musculoskeletal condition of any of the preceding claims wherein said dosage form demonstrates improved drug dissolution rates as compared to the metaxalone product of NDA #13-217, wherein dissolution rates are measured 30 minutes after the pharmaceutical solid dosage form or the metaxalone product of NDA #13-217 is placed in

(i) a peak glass dissolution vessel filled with 1000 mL of purified water, maintained at 25°C and stirred at a paddle speed of 100 rpm using a USP Type II (paddle) apparatus; or

(ii) a standard glass dissolution vessel filled with 1000 mL of purified water, maintained at 35°C and stirred at a paddle speed of 100 rpm using a USP Type II (paddle) apparatus; or

(iii) a peak glass dissolution vessel filled with 500 mL of an aqueous solution of 0.1 % by weight of Sodium Lauryl Sulfate per volume of water, maintained at 37°C and stirred at a paddle speed of 50 rpm using a USP Type II (paddle) apparatus.

**Patentansprüche**

1. Eine pharmazeutische feste Dosisform, die Metaxalon, wenigstens einen Pulverarzneiträger, der ausgewählt ist aus einer Gruppe, bestehend aus kaum wasserlöslichen oder im Wesentlichen wasserunlöslichen natürlichen oder synthetischen Hydrokolloiden und Polymeren, und wenigstens eine nicht flüchtige Flüssigkeit umfasst, die hergestellt wird durch ein Verfahren, das die folgenden Schritte umfasst:

das Bereitstellen einer wirksamen Menge von Metaxaion;

das Bereitstellen wenigstens eines Pulverarzneiträgers, der ausgewählt ist aus der Gruppe, bestehend aus Natriumalginat, Ammoniumalginat, Natriumcarboxymethylcellulose, mikrokristalliner Cellulose, vorgelierter Stärke, Natrium-Stärke-Glycolat, Methacrylsäurepolymeren oder Kombinationen von diesen;

das trockene Mischen der wirksamen Menge an Metaxalon und des wenigstens einen Pulverarzneiträgers, der ausgewählt ist aus der Gruppe, bestehend aus Natriumalginat, Ammoniumalginat, Natriumcarboxymethylcellulose, mikrokristalliner Cellulose, vorgelierter Stärke, Natrium-Stärke-Glycolat, Methacrylsäurepolymeren oder Kombinationen von diesen;

das Anfeuchten der Metaxalon/Pulver-Mischung mit der wenigstens einen nicht flüchtigen Flüssigkeit und wenigstens einer flüchtigen Flüssigkeit, um eine Mischung zu bilden;

das Granulieren der Mischung, um ein Granulat zu bilden;

das Trocknen und Mahlen des Granulats;

das Mischen mit wenigstens einem anderen inaktiven Pulverarzneiträger;

und das Komprimieren zu Tabletten, um die pharmazeutische feste Dosisform herzustellen;

wobei die wenigstens eine nicht flüchtige Flüssigkeit ausgewählt ist aus der Gruppe, bestehend aus Propylenglycol, Glycerin, flüssigen Polyethylenglycolen, halbfesten Polyethylenglycolen, Pharmasolve, flüssigen Polysorbaten, halbfesten Polysorbaten, flüssigen Spannmitteln (spans), halbfesten Spannmitteln, flüssigen Cremophoren, halbfesten Cremophoren, flüssigen Pluronica, halbfesten Pluronica, flüssigen Myglyolen, halbfesten Myglyolen, nicht flüchtigen Ölen, Vitamin E, Lecithin oder Kombinationen von diesen;

die wenigstens eine flüchtige Flüssigkeit ausgewählt ist aus der Gruppe, bestehend aus Methanol, Ethanol, Aceton, Wasser oder Kombinationen von diesen;

und wobei:

(i) 29 Gew.-% oder mehr des Metaxalons nach ca. 30 Minuten gelöst sind, nachdem die Dosisform in ein spitz zulaufendes (peak) Glasauflösungsgefäß gegeben wurde, das mit 1000 ml gereinigten Wasser gefüllt war, welches bei 25°C gehalten wurde und bei einer Blattgeschwindigkeit von 100 UpM unter Verwendung eines USP Typ II (Blatt-)Geräts gerührt wurde; oder

(ii) 40 Gew.-% oder mehr des Metaxalons nach ca. 30 Minuten gelöst sind, nachdem die Dosisform in ein Standard-Glasauflösungsgefäß gegeben wurde, das mit 1000 ml gereinigten Wasser gefüllt war, welches bei 35°C gehalten wurde und bei einer Blattgeschwindigkeit von 100 UpM unter Verwendung eines USP Typ II (Blatt-)Geräts gerührt wurde; oder

(iii) 30 Gew.-% oder mehr des Metaxalons nach ca. 30 Minuten gelöst sind, nachdem die Dosisform in ein spitz zulaufendes Glasauflösungsgefäß gegeben wurde, das mit 500 ml einer wässrigen Lösung von 0,1 Gew.-% Natriumlaurylsulfat pro Volumen Wasser gefüllt war, welches bei 37°C gehalten wurde und bei einer Blattgeschwindigkeit von 50 UpM unter Verwendung eines USP Typ II (Blatt-)Geräts gerührt wurde.

2. Die pharmazeutische feste Dosisform gemäß Anspruch 1, wobei:

(i) 34 Gew.-% oder mehr des Metaxalons nach ca. 30 Minuten gelöst sind, nachdem die Dosisform in ein spitz zulaufendes Glasauflösungsgefäß gegeben wurde, das mit 1000 ml gereinigten Wasser gefüllt war, welches bei 25°C gehalten wurde und bei einer Blattgeschwindigkeit von 100 UpM unter Verwendung eines USP Typ II (Blatt-)Geräts gerührt wurde; oder

(ii) 48 Gew.-% oder mehr des Metaxalons nach ca. 30 Minuten gelöst sind, nachdem die Dosisform in ein Standard-Glasauflösungsgefäß gegeben wurde, das mit 1000 ml gereinigten Wasser gefüllt war, welches bei 35°C gehalten wurde und bei einer Blattgeschwindigkeit von 100 UpM unter Verwendung eines USP Typ II (Blatt-)Geräts gerührt wurde; oder

(iii) 37 Gew.-% oder mehr des Metaxalons nach ca. 30 Minuten gelöst sind, nachdem die Dosisform in ein spitz zulaufendes Glasauflösungsgefäß gegeben wurde, das mit 500 ml einer wässrigen Lösung von 0,1 Gew.-% Natriumlaurylsulfat pro Volumen Wasser gefüllt war, welches bei 37°C gehalten wurde und bei einer Blattgeschwindigkeit von 50 UpM unter Verwendung eines USP Typ II (Blatt-)Geräts gerührt wurde.

3. Die pharmazeutische feste Dosisform gemäß Anspruch 1, wobei:

(i) 39 Gew.-% oder mehr des Metaxalons nach ca. 30 Minuten gelöst sind, nachdem die Dosisform in ein spitz zulaufendes Glasauflösungsgefäß gegeben wurde, das mit 1000 ml gereinigten Wasser gefüllt war, welches bei 25°C gehalten wurde und bei einer Blattgeschwindigkeit von 100 UpM unter Verwendung eines USP Typ II (Blatt-)Geräts gerührt wurde; oder

(ii) 52 Gew.-% oder mehr des Metaxalons nach ca. 30 Minuten gelöst sind, nachdem die Dosisform in ein Standard-Glasauflösungsgefäß gegeben wurde, das mit 1000 ml gereinigten Wasser gefüllt war, welches bei 35°C gehalten wurde und bei einer Blattgeschwindigkeit von 100 UpM unter Verwendung eines USP Typ II (Blatt-)Geräts gerührt wurde; oder

(iii) 42 Gew.-% oder mehr des Metaxalons nach ca. 30 Minuten gelöst sind, nachdem die Dosisform in ein spitz zulaufendes Glasauflösungsgefäß gegeben wurde, das mit 500 ml einer wässrigen Lösung von 0,1 Gew.-% Natriumlaurylsulfat pro Volumen Wasser gefüllt war, welches bei 37°C gehalten wurde und bei einer Blattgeschwindigkeit von 50 UpM unter Verwendung eines USP Typ II (Blatt-)Geräts gerührt wurde.

4. Die pharmazeutische feste Dosisform gemäß Anspruch 1, wobei:

(i) 46 Gew.-% oder mehr des Metaxalons nach ca. 30 Minuten gelöst sind, nachdem die Dosisform in ein spitz zulaufendes Glasauflösungsgefäß gegeben wurde, das mit 1000 ml gereinigten Wasser gefüllt war, welches bei 25°C gehalten wurde und bei einer Blattgeschwindigkeit von 100 UpM unter Verwendung eines USP Typ II (Blatt-)Geräts gerührt wurde; oder

(ii) 57 Gew.-% oder mehr des Metaxalons nach ca. 30 Minuten gelöst sind, nachdem die Dosisform in ein Standard-Glasauflösungsgefäß gegeben wurde, das mit 1000 ml gereinigten Wasser gefüllt war, welches bei 35°C gehalten wurde und bei einer Blattgeschwindigkeit von 100 UpM unter Verwendung eines USP Typ II (Blatt-)Geräts gerührt wurde; oder

(iii) 48 Gew.-% oder mehr des Metaxalons nach ca. 30 Minuten gelöst sind, nachdem die Dosisform in ein spitz zulaufendes Glasauflösungsgefäß gegeben wurde, das mit 500 ml einer wässrigen Lösung von 0,1 Gew.-% Natriumlaurylsulfat pro Volumen Wasser gefüllt war, welches bei 37°C gehalten wurde und bei einer Blattgeschwindigkeit von 50 UpM unter Verwendung eines USP Typ II (Blatt-)Geräts gerührt wurde.

5. Die pharmazeutische feste Dosisform gemäß irgendeinem vorhergehenden Anspruch, wobei die Dosisform nach oraler Verabreichung an fastende oder nicht fastende menschliche Patienten bioäquivalent ist zu oder stärker bioverfügbar ist als das Metaxalonprodukt von NDA #13-217.

6. Die pharmazeutische feste Dosisform gemäß irgendeinem der Ansprüche 1 bis 4, wobei die Dosisform nach oraler Verabreichung an fastende oder nicht fastende menschliche Patienten stärker bioverfügbar ist als das Metaxalonprodukt von NDA #13-217.

7. Die pharmazeutische feste Dosisform gemäß irgendeinem vorhergehenden Anspruch, wobei die Dosisform im Vergleich zu dem Metaxalonprodukt von NDA #13-217 verbesserte Arzneimittelauflösungsraten zeigt, wobei die Auflösungsraten nach 30 Minuten gemessen wurden, nachdem die pharmazeutische feste Dosisform oder das Metaxalonprodukt von NDA #13-217 gegeben wurden in

(i) ein spitz zulaufendes Glasauflösungsgefäß, das mit 1000 ml gereinigten Wasser gefüllt war, welches bei 25°C gehalten wurde und bei einer Blattgeschwindigkeit von 100 UpM unter Verwendung eines USP Typ II (Blatt-)Geräts gerührt wurde; oder

(ii) ein Standard-Glasauflösungsgefäß, das mit 1000 ml gereinigten Wasser gefüllt war, welches bei 35°C gehalten wurde und bei einer Blattgeschwindigkeit von 100 UpM unter Verwendung eines USP Typ II (Blatt-)Geräts gerührt wurde; oder

(iii) ein spitz zulaufendes Glasauflösungsgefäß, das mit 500 ml einer wässrigen Lösung von 0,1 Gew.-% Natriumlaurylsulfat pro Volumen Wasser gefüllt war, welches bei 37°C gehalten wurde und bei einer Blattgeschwindigkeit von 50 UpM unter Verwendung eines USP Typ II (Blatt-)Geräts gerührt wurde.

8. Eine pharmazeutische feste Dosisform gemäß irgendeinem vorhergehenden Anspruch zur Verwendung bei der Behandlung einer muskuloskeletalen Erkrankung.

9. Ein Verfahren zur Erzeugung einer pharmazeutischen festen Dosisform gemäß irgendeinem der vorhergehenden Ansprüche, umfassend:

das Bereitstellen einer wirksamen Menge von Metaxaion;
das Bereitstellen wenigstens eines Pulverarzneiträgers, der ausgewählt ist aus der Gruppe, bestehend aus Natriumalginat, Ammoniumalginat, Natriumcarboxymethylcellulose, mikrokristalliner Cellulose, vorgelierter Stärke, Natrium-Stärke-Glycolat, Methacrylsäurepolymeren oder Kombinationen von diesen;

das trockene Mischen der wirksamen Menge an Metaxalon und des wenigstens einen Pulverarzneiträgers, der ausgewählt ist aus der Gruppe, bestehend aus Natriumalginat, Ammoniumalginat, Natriumcarboxymethylcellulose, mikrokristalliner Cellulose, vorgelierter Stärke, Natrium-Stärke-Glycolat, Methacrylsäurepolymeren oder Kombinationen von diesen;

das Anfeuchten der Metaxalon/Pulver-Mischung mit der wenigstens einen nicht flüchtigen Flüssigkeit und wenigstens einer flüchtigen Flüssigkeit, um eine Mischung zu bilden;

das Granulieren der Mischung, um ein Granulat zu bilden;

das Trocknen und Mahlen des Granulats;

das Mischen mit wenigstens einem anderen Pulverarzneiträger;

und das Komprimieren zu Tabletten, um die pharmazeutische feste Dosisform herzustellen;

wobei die wenigstens eine nicht flüchtige Flüssigkeit ausgewählt wird aus der Gruppe, bestehend aus Propylenglycol, Glycerin, flüssigen Polyethylenglycolen, halbfesten Polyethylenglycolen, Pharmasolve, flüssigen Polysorbaten, halbfesten Polysorbaten, flüssigen Spannmitteln, halbfesten Spannmitteln, flüssigen Cremophoren, halbfesten Cremophoren, flüssigen Pluronica, halbfesten Pluronica, flüssigen Myglyolen, halbfesten Myglyolen, nicht flüchtigen Ölen, Vitamin E, Lecithin oder Kombinationen von diesen; und

die wenigstens eine flüchtige Flüssigkeit ausgewählt wird aus der Gruppe, bestehend aus Methanol, Ethanol, Aceton, Wasser oder Kombinationen von diesen.

10. Ein Verfahren zur Erzeugung einer pharmazeutischen festen Dosisform zur Behandlung einer muskuloskeletalen Erkrankung nach irgendeinem der vorhergehenden Ansprüche, wobei die Dosisform nach oraler Verabreichung an fastende oder nicht fastende menschliche Patienten bioäquivalent ist zu oder stärker bioverfügbar ist als das Metaxalonprodukt von NDA #13-217.

11. Ein Verfahren zur Erzeugung einer pharmazeutischen festen Dosisform zur Behandlung einer muskuloskeletalen Erkrankung nach irgendeinem der vorhergehenden Ansprüche, wobei die Dosisform nach oraler Verabreichung an fastende oder nicht fastende menschliche Patienten stärker bioverfügbar ist als das Metaxalonprodukt von NDA #13-217.

12. Ein Verfahren zur Erzeugung einer pharmazeutischen festen Dosisform zur Behandlung einer muskuloskeletalen Erkrankung nach irgendeinem der vorhergehenden Ansprüche, wobei die Dosisform im Vergleich zu dem Metaxalonprodukt von NDA #13-217 verbesserte Arzneimittelauflösungsraten zeigt, wobei die Auflösungsraten nach 30 Minuten gemessen wurden, nachdem die pharmazeutische feste Dosisform oder das Metaxalonprodukt von NDA #13-217 gegeben wurden in

(i) ein spitz zulaufendes Glasauflösungsgefäß, das mit 1000 ml gereinigten Wasser gefüllt war, welches bei 25°C gehalten wurde und bei einer Blattgeschwindigkeit von 100 UpM unter Verwendung eines USP Typ II (Blatt-)Geräts gerührt wurde; oder

(ii) ein Standard-Glasauflösungsgefäß, das mit 1000 ml gereinigten Wasser gefüllt war, welches bei 35°C gehalten wurde und bei einer Blattgeschwindigkeit von 100 UpM unter Verwendung eines USP Typ II (Blatt-) Geräts gerührt wurde; oder

(iii) ein spitz zulaufendes Glasauflösungsgefäß, das mit 500 ml einer wässrigen Lösung von 0,1 Gew.-% Natriumlaurylsulfat pro Volumen Wasser gefüllt war, welches bei 37°C gehalten wurde und bei einer Blattgeschwindigkeit von 50 UpM unter Verwendung eines USP Typ II (Blatt-)Geräts gerührt wurde.

## Revendications

1. Forme pharmaceutique solide comprenant de la métaxalone, au moins un excipient en poudre choisi dans un groupe comprenant des hydrocolloïdes et polymères naturels ou synthétiques, peu hydrosoluble ou pratiquement insoluble dans l'eau, et au moins un liquide non volatil, préparée selon un procédé comprenant les étapes de :

fourniture d'une quantité efficace de métaxalone ;

fourniture d'au moins un excipient en poudre choisi dans le groupe constitué d'alginate de sodium, d'alginate d'ammonium, de carboxyméthylcellulose sodique, de cellulose microcristalline, d'amidon prégélatinisé, de glycolate d'amidon sodique, de copolymères d'acide méthacrylique, ou de combinaisons de ceux-ci ;

mélange à sec de la quantité efficace de métaxalone et du le au moins un excipient en poudre choisi dans le groupe constitué d'alginate de sodium, d'alginate d'ammonium, de carboxyméthylcellulose sodique, de cellulose microcristalline, d'amidon prégélatinisé, de glycolate d'amidon sodique, de copolymères d'acide méthacrylique, ou de combinaisons de ceux-ci ;

mouillage du mélange de métaxalone/poudre avec le au moins un liquide non volatil et au moins un liquide volatil pour former un mélange ;

granulation du mélange pour former une granulation ;

séchage et broyage de la granulation ;

mélange avec au moins un autre excipient en poudre inactif et compression en comprimés pour produire la forme pharmaceutique solide ;

le au moins un liquide non volatil étant choisi dans le groupe constitué de propylène glycol, de glycérine, de polyéthylènes glycols liquides, de polyéthylènes glycols semi-solides, de Pharmasolve, de polysorbates liquides, de polysorbates semi-solides, de Span liquides, de Span semi-solides, de Cremophor liquides, de Cremophor semi-solides, de Pluronic liquides, de Pluronic semi-solides, de Miglyol liquides, de Miglyol semi-solides, d'huiles non volatiles, de vitamine E, de lécithine, ou de combinaisons de ceux-ci ;

le au moins un liquide volatil étant choisi dans le groupe constitué de méthanol, d'éthanol, d'acétone, d'eau, ou de combinaisons de ceux-ci ; et où :

(i) 29 % en poids ou plus de ladite métaxolone est dissoute environ 30 minutes après que ladite forme pharmaceutique ait été placée dans une cuve de dissolution en verre Peak remplie avec 1000 ml d'eau purifiée, maintenue à 25 °C et agitée à une vitesse de pales de 100 tours/min en utilisant un appareil USP de type II (à pales) ; ou

(ii) 40 % en poids ou plus de ladite métaxalone est dissoute environ 30 minutes après que ladite forme pharmaceutique ait été placée dans une cuve de dissolution en verre standard remplie avec 1000 ml d'eau purifiée, maintenue à 35 °C et agitée à une vitesse de pales de 100 tours/min en utilisant un appareil USP de type II (à pales) ; ou

(iii) 30 % en poids ou plus de ladite métaxalone est dissoute environ 30 minutes après que ladite forme pharmaceutique ait été placée dans une cuve de dissolution en verre Peak remplie avec 500 ml d'une solution aqueuse de 0,1 % en poids de laurylsulfate de sodium par volume d'eau, maintenue à 37 °C et agitée à une vitesse de pales de 50 tours/min en utilisant un appareil USP de type II (à pales).

2. Forme pharmaceutique solide selon la revendication 1, dans laquelle :

(i) 34 % en poids ou plus de ladite métaxalone est dissoute environ 30 minutes après que ladite forme pharmaceutique ait été placée dans une cuve de dissolution en verre Peak remplie avec 1000 ml d'eau purifiée, maintenue à 25 °C et agitée à une vitesse de pales de 100 tours/min en utilisant un appareil USP de type II (à pales) ; ou

(ii) 48 % en poids ou plus de ladite métaxalone est dissoute environ 30 minutes après que ladite forme pharmaceutique ait été placée dans une cuve de dissolution en verre standard remplie avec 1000 ml d'eau purifiée, maintenue à 35 °C et agitée à une vitesse de pales de 100 tours/min en utilisant un appareil USP de type II (à pales) ; ou

(iii) 37 % en poids ou plus de ladite métaxalone est dissoute environ 30 minutes après que ladite forme pharmaceutique ait été placée dans une cuve de dissolution en verre Peak remplie avec 500 ml d'une solution aqueuse de 0,1 % en poids de laurylsulfate de sodium par volume d'eau, maintenue à 37 °C et agitée à une vitesse de pales de 50 tours/min en utilisant un appareil USP de type II (à pales).

3. Forme pharmaceutique solide selon la revendication 1, dans laquelle :

(i) 39 % en poids ou plus de ladite métaxalone est dissoute environ 30 minutes après que ladite forme pharmaceutique ait été placée dans une cuve de dissolution en verre Peak remplie avec 1000 ml d'eau purifiée, maintenue à 25 °C et agitée à une vitesse de pales de 100 tours/min en utilisant un appareil USP de type II (à pales) ; ou

(ii) 52 % en poids ou plus de ladite métaxalone est dissoute environ 30 minutes après que ladite forme pharmaceutique ait été placée dans une cuve de dissolution en verre standard remplie avec 1000 ml d'eau purifiée, maintenue à 35 °C et agitée à une vitesse de pales de 100 tours/min en utilisant un appareil USP de type II (à pales) ; ou

(iii) 42 % en poids ou plus de ladite métaxalone est dissoute environ 30 minutes après que ladite forme pharmaceutique ait été placée dans une cuve de dissolution en verre Peak remplie avec 500 ml d'une solution aqueuse de 0,1 % en poids de laurylsulfate de sodium par volume d'eau, maintenue à 37 °C et agitée à une vitesse de pales de 50 tours/min en utilisant un appareil USP de type II (à pales).

4. Forme pharmaceutique solide selon la revendication 1, dans laquelle :

(i) 46 % en poids ou plus de ladite métaxalone est dissoute environ 30 minutes après que ladite forme pharmaceutique ait été placée dans une cuve de dissolution en verre Peak remplie avec 1000 ml d'eau purifiée, maintenue à 25 °C et agitée à une vitesse de pales de 100 tours/min en utilisant un appareil USP de type II (à pales) ; ou

(ii) 57 % en poids ou plus de ladite métaxalone est dissoute environ 30 minutes après que ladite forme pharmaceutique ait été placée dans une cuve de dissolution en verre standard remplie avec 1000 ml d'eau purifiée, maintenue à 35 °C et agitée à une vitesse de pales de 100 tours/min en utilisant un appareil USP de type II (à pales) ; ou

(iii) 48 % en poids ou plus de ladite métaxalone est dissoute environ 30 minutes après que ladite forme pharmaceutique ait été placée dans une cuve de dissolution en verre Peak remplie avec 500 ml d'une solution aqueuse de 0,1 % en poids de laurylsulfate de sodium par volume d'eau, maintenue à 37 °C et agitée à une vitesse de pales de 50 tours/min en utilisant un appareil USP de type II (à pales).

5. Forme pharmaceutique solide selon l'une quelconque des revendications précédentes, la forme pharmaceutique étant bioéquivalente à ou plus biodisponible que le produit de métaxalone de NDA n° 13-217 après une administration orale à des sujets humains à jeun ou non à jeun.

6. Forme pharmaceutique solide selon l'une quelconque des revendications 1 à 4, la forme pharmaceutique étant plus biodisponible que le produit de métaxalone de NDA n° 13-217 après une administration orale à des sujets humains à jeun ou non à jeun.

7. Forme pharmaceutique solide selon l'une quelconque des revendications précédentes, la forme pharmaceutique présentant des taux de dissolution de médicament améliorés par rapport au produit de métaxalone de NDA n° 13-217, les taux de dissolution étant mesurés 30 minutes après que la forme pharmaceutique solide ou le produit de métaxalone de NDA n° 13-217 ait été placé dans

(i) une cuve de dissolution en verre Peak remplie avec 1000 ml d'eau purifiée, maintenue à 25 °C et agitée à une vitesse de pales de 100 tours/min en utilisant un appareil USP de type II (à pales) ; ou

(ii) une cuve de dissolution en verre standard remplie avec 1000 ml d'eau purifiée, maintenue à 35 °C et agitée à une vitesse de pales de 100 tours/min en utilisant un appareil USP de type II (à pales) ; ou

(iii) une cuve de dissolution en verre Peak remplie avec 500 ml d'une solution aqueuse de 0,1 % en poids de laurylsulfate de sodium par volume d'eau, maintenue à 37 °C et agitée à une vitesse de pales de 50 tours/min en utilisant un appareil USP de type II (à pales).

8. Forme pharmaceutique solide selon l'une quelconque des revendications précédentes, pour une utilisation dans le traitement d'une affection musculosquelettique.

9. Procédé de fabrication d'une forme pharmaceutique solide selon l'une quelconque des revendications précédentes, comprenant :

la fourniture d'une quantité efficace de métaxalone ;
la fourniture d'au moins un excipient en poudre choisi dans le groupe constitué d'alginate de sodium, d'alginate d'ammonium, de carboxyméthylcellulose sodique, de cellulose microcristalline, d'amidon prégélatinisé, de glycolate d'amidon sodique, de copolymères d'acide méthacrylique, ou de combinaisons de ceux-ci ;
le mélange à sec de la quantité efficace de métaxalone et du le au moins un excipient en poudre choisi dans le groupe constitué d'alginate de sodium, d'alginate d'ammonium, de carboxyméthylcellulose sodique, de cellulose microcristalline, d'amidon prégélatinisé, de glycolate d'amidon sodique, de copolymères d'acide méthacrylique, ou de combinaisons de ceux-ci ;
le mouillage du mélange de métaxalone/poudre avec le au moins un liquide non volatil et au moins un liquide volatil pour former un mélange ;
la granulation du mélange pour former une granulation ;
le séchage et le broyage de la granulation ;
le mélange avec au moins un autre excipient en poudre ;
et la compression en comprimés pour produire la forme pharmaceutique solide ;

le au moins un liquide non volatil étant choisi dans le groupe constitué de propylène glycol, de glycérine, de polyéthylènes glycols liquides, de polyéthylènes glycols semi-solides, de Pharmasolve, de polysorbates liquides, de polysorbates semi-solides, de Span liquides, de Span semi-solides, de Cremophor liquides, de Cremophor semi-solides, de Pluronic liquides, de Pluronic semi-solides, de Miglyol liquides, de Miglyol semi-solides, d'huiles non volatiles, de vitamine E, de lécithine, ou de combinaisons de ceux-ci ;

le au moins un liquide volatil étant choisi dans le groupe constitué de méthanol, d'éthanol, d'acétone, d'eau, ou de combinaisons de ceux-ci.

10. Procédé de fabrication d'une forme pharmaceutique solide pour traiter une affection musculosquelettique selon l'une quelconque des revendications précédentes, ladite forme pharmaceutique étant bioéquivalente à ou plus biodisponible que le produit de métaxalone de NDA n° 13-217 après une administration orale à des sujets humains à jeun ou non à jeun.

11. Procédé de fabrication d'une forme pharmaceutique solide pour traiter une affection musculosquelettique selon l'une quelconque des revendications précédentes, ladite forme pharmaceutique étant plus biodisponible que le produit de métaxalone de NDA n° 13-217 après une administration orale à des sujets humains à jeun ou non à jeun.

12. Procédé de fabrication d'une forme pharmaceutique solide pour traiter une affection musculosquelettique selon l'une quelconque des revendications précédentes, ladite forme pharmaceutique présentant des taux de dissolution de médicament améliorés par rapport au produit de métaxalone de NDA n° 13-217, les taux de dissolution étant mesurés 30 minutes après que la forme pharmaceutique solide ou le produit de métaxalone de NDA n° 13-217 ait été placé dans

(i) une cuve de dissolution en verre Peak remplie avec 1000 ml d'eau purifiée, maintenue à 25 °C et agitée à une vitesse de pales de 100 tours/min en utilisant un appareil USP de type II (à pales) ; ou
(ii) une cuve de dissolution en verre standard remplie avec 1000 ml d'eau purifiée, maintenue à 35 °C et agitée à une vitesse de pales de 100 tours/min en utilisant un appareil USP de type II (à pales) ; ou
(iii) une cuve de dissolution en verre Peak remplie avec 500 ml d'une solution aqueuse de 0,1 % en poids de laurylsulfate de sodium par volume d'eau, maintenue à 37 °C et agitée à une vitesse de pales de 50 tours/min en utilisant un appareil USP de type II (à pales).

## A RELATIVE BIOAVAILABILITY STUDY OF 400 MG METAXALONE TABLETS UNDER NON-FASTING CONDITIONS

### Ln-Transformed Data

| PK Variable | Least Squares Mean | | Geometric Mean | | | 90 % Confidence Interval | P-values for Product Effects | Power of ANOVA | ANOVA % CV |
|---|---|---|---|---|---|---|---|---|---|
| | Test | Reference | Test | Reference | % Ratio | (Lower Limit, Upper Limit) | | | |
| $C_{max}$ | 6.817 | 6.890 | 912.99 | 981.95 | 92.98 | (74.66, 115.78) | 0.5744 | 0.3863 | 46.51% |
| $AUC_{0-t}$ | 8.608 | 8.712 | 5475.82 | 6077.07 | 90.11 | (82.12, 98.86) | 0.0668 | 0.9764 | 18.88% |
| $AUC_{inf}$ | 8.641 | 8.782 | 5659.68 | 6515.93 | 86.86 | (79.95, 94.36) | 0.0079 | 0.9930 | 16.83% |

### Non-Transformed Data

| PK Variable | Least Squares Mean | | | 90 % Confidence Interval | P-values for Product Effects | Power of ANOVA |
|---|---|---|---|---|---|---|
| | Test | Reference | % Ratio | (Lower Limit, Upper Limit) | | |
| $C_{max}$ | 1049.52 | 1228.82 | 85.41 | (66.11, 104.71) | 0.2077 | 0.3982 |
| $AUC_{0-t}$ | 6018.48 | 6724.43 | 89.50 | (79.26, 99.74) | 0.0921 | 0.8935 |
| $AUC_{inf}$ | 6225.74 | 7125.24 | 87.38 | (78.16, 96.59) | 0.0280 | 0.9453 |
| $T_{max}$ | 6.65 | 7.98 | 83.33 | (52.35, 114.31) | 0.3656 | 0.1855 |
| $k_{elim}$ | 0.1773 | 0.2251 | 78.78 | (57.52, 100.04) | 0.1005 | 0.3394 |
| $T_{1/2}$ | 4.72 | 4.92 | 95.98 | (63.29, 128.68) | 0.8348 | 0.1713 |

# FIGURE 1

# A RELATIVE BIOAVAILABILITY STUDY OF 400 MG METAXALONE TABLETS UNDER FASTING CONDITIONS

### Ln-Transformed Data

| PK Variable | Least Squares Mean | | Geometric Mean | | | 90 % Confidence Interval | P-values for Product Effects | Power of ANOVA | ANOVA %CV |
|---|---|---|---|---|---|---|---|---|---|
| | Test | Reference | Test | Reference | % Ratio | (Lower Limit, Upper Limit) | | | |
| $C_{max}$ | 6.473 | 6.406 | 647.37 | 605.54 | 106.91 | (95.78, 119.33) | 0.3122 | 0.9157 | 30.97% |
| $AUC_{0-t}$ | 8.359 | 8.492 | 4266.73 | 4875.73 | 87.51 | (82.6, 92.71) | 0.0004 | 1.0000 | 16.01% |
| $AUC_{inf}$ | 8.416 | 8.538 | 4518.25 | 5107.39 | 88.47 | (83.49, 93.74) | 0.0009 | 1.0000 | 16.05% |

### Non-Transformed Data

| PK Variable | Least Squares Mean | | | 90 % Confidence Interval | P-values for Product Effects | Power of ANOVA |
|---|---|---|---|---|---|---|
| | Test | Reference | % Ratio | (Lower Limit, Upper Limit) | | |
| $C_{max}$ | 709.73 | 671.93 | 105.63 | (94.85, 116.41) | 0.3849 | 0.8618 |
| $AUC_{0-t}$ | 4727.87 | 5381.28 | 87.86 | (81.68, 94.04) | 0.0020 | 0.9996 |
| $AUC_{inf}$ | 4989.63 | 5631.53 | 88.60 | (82.61, 94.59) | 0.0026 | 0.9998 |
| $T_{max}$ | 3.50 | 4.07 | 86.09 | (70.1, 102.08) | 0.1507 | 0.5381 |
| $k_{el}$ | 0.1178 | 0.1324 | 88.96 | (74.32, 103.6) | 0.2115 | 0.6124 |
| $t_{1/2}$ | 7.58 | 6.13 | 123.51 | (106.58, 140.43) | 0.0244 | 0.4929 |

# FIGURE 2

Mean Plasma Concentration (0 - 36 hours) Under Fasting Conditions
N=43

**FIGURE 3**

FIGURE 4

## Mean Plasma Concentration of Metaxalone:

Obtained After Single Oral Administration to Fasting Healthy Male
Volunteers (N=8) of 400 mg Metaxalone Test and Reference Tablets

—●— Test: Ex.#4 Metaxaolone 400-mg Tablets (Lot No. BB5800056)

—□— Reference: Skelaxin 400-mg Tablets (Lot No. GS803A)

## FIGURE 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6407128 B
- US 6683102 B
- WO 2004019937 A
- US 5800834 A
- US 5968550 A
- US 6096337 A
- US 6423339 B

**Non-patent literature cited in the description**

- Remington: The Science and Practice of Pharmacy. 2000
- Theory & Practice of Industrial Pharmacy. Lea & Febiger